# EUROPEAN PATENT SPECIFICATION

(11) **EP 1 246 835 B1**
(45) Date of publication and mention of the grant of the patent: **07.01.2009**
(21) Application number: 01900185.8
(22) Date of filing: 08.01.2001
(51) Int. Cl.: C07J 1/00, A61K 31/58

(54) **SUBSTITUTED SAPOGENINS AND THEIR USE**
SUBSTITUIERTE SAPOGENINE UND IHRE VERWENDUNG
SAPOGENINES SUBSTITUEES ET LEUR UTILISATION

(30) Priority: 06.01.2000 GB 0000228
(43) Date of publication of application: 09.10.2002
(73) Proprietor: PHYTOPHARM PLC, Godmanchester, Cambridgeshire PE18 8HG (GB)
(72) Inventor: BARRACLOUGH, Paul, Maidstone, Kent ME15 9SB (GB); HANSON, Jim, Steyning, West Sussex BN44 3PF (GB); GUNNING, Phil, Grantchester, Cambridgeshire CB3 9NP (GB); REES, Daryl, Sandy SG19 2DD (GB); XIA, Zongqin, Shanghai 200025 (CN); HU, Yaer, Shanghai 200025 (CN)
(74) Representative: Brown, David Leslie
(86) International application number: PCT/GB2001/000048
(87) International publication number: WO 2001/049703

(56) References cited:
- EP-A- 1 024 146
- WO-A-99/48482
- WO-A-99/48507
- DE-A- 4 303 214
- KINOSHITA KAORU ET AL: "Antinociceptive effect of triterpenes from cacti." PHARMACEUTICAL BIOLOGY, vol. 36, no. 1, January 1998 (1998-01), pages 50-57, XP002168408 ISSN: 1388-0209
- DATABASE CHEMABS [Online] CHEMICAL ABSTRACTS SERVICE, COLUMBUS, OHIO, US; YI, NINGYU ET AL: "Extraction of.beta.-adrenergic receptor- and M cholinergic receptor-regulating (.beta.,5.beta.,25s)-spirostan-3-ol from Anemarrhena asphodeloides Bunge" retrieved from STN Database accession no. 123:266081 XP002168420 & CN 1 096 031 A (SHANGHAI NO. 2 MEDICAL UNIVERSITY, PEOP. REP. CHINA) 7 December 1994 (1994-12-07)
- N YI ET AL: "Sarsasapogenin: Mechanism in treating senile dementia" SYNTHESIS AND APPLICATIONS OF ISOTOPICALLY LABELLED COMPOUNDS,XX,XX, 14 September 1997 (1997-09-14), pages 315-320, XP002117140
- CAMBIAGHI S ET AL: "STRUCTURE AND ABSOLUTE CONFIGURATION OF NEW ALKALOIDS FROM SOLANUM-PANICULATUM-D" ANNALI DI CHIMICA, vol. 61, no. 1, 1971, pages 99-111, XP002168409 ISSN: 0003-4592
- K. SCHREIBER ET AL: "Jurubine, a novel type of steroidal saponin with (25S)-3.beta.-amino-5.alpha.-furostane-22. alpha.,26-diol O(26)-beta-D glucopyranoside structure from Solanum paniculatum l." TETRAHEDRON LETTERS., no. 48, 1966, pages 5997-6002, XP002168410 ELSEVIER SCIENCE PUBLISHERS, AMSTERDAM., NL ISSN: 0040-4039
- M. S. BEDOUR ET AL: "Steroid Sapogenins VII. Identification and Origin of 25D-Spirosta-3,5-dien Among the Fenugreek Sapogenins" JOURNAL OF PHARMACEUTICAL SCIENCES., vol. 53, no. 10, October 1964 (1964-10), pages 1276-1278, XP002168411 AMERICAN PHARMACEUTICAL ASSOCIATION. WASHINGTON., US ISSN: 0022-3549
- BARTON, DEREK H. R. ET AL: "Reactions of relevance to the chemistry of aminoglycoside antibiotics. Part 14. A useful radical-deamination reaction" J. CHEM. SOC., PERKIN TRANS. 1 (1980), (12), 2657-64 , XP002168412
- DATABASE BIOSIS [Online] BIOSCIENCES INFORMATION SERVICE, PHILADELPHIA, PA, US; 1983 CHAKRAVARTY A K ET AL: "JURIPIDINE A 3 AMINO STEROIDAL ALKALOID FROM ROOTS OF SOLANUM-HISPIDUM" Database accession no. PREV198478054691 XP002168421 & PHYTOCHEMISTRY (OXFORD), vol. 22, no. 12, 1983, pages 2843-2846, ISSN: 0031-9422
- N. L. WENDLER ET AL: "The Transformation of Manogenin to Hecogenin" JOURNAL OF THE AMERICAN CHEMICAL SOCIETY., vol. 74, no. 19, 5 October 1952 (1952-10-05), pages 4894-4897, XP002168413 AMERICAN CHEMICAL SOCIETY, WASHINGTON, DC., US ISSN: 0002-7863
- DENINNO, MICHAEL P.: ""Anomalous" Ozonolysis of Cyclic Allylic Alcohols: Mechanism and Synthetic Utility" J. AM. CHEM. SOC. (1995), 117(39), 9927-8 , XP002168414
- K. TAKEDA ET AL: "Studies on the Steroidal Components of Domestic Plants. Part LIV. Synthesis of Isodiotigenin" JOURNAL OF THE CHEMICAL SOCIETY, SECTION C: ORGANIC CHEMISTRY., 1968, pages 1041-1044, XP002168415 CHEMICAL SOCIETY. LETCHWORTH., GB ISSN: 1472-7781
- K. TAKEDA ET AL: "The Structure of Metagenin" TETRAHEDRON LETTERS., no. 3, 1960, page 1-8 XP002168416 ELSEVIER SCIENCE PUBLISHERS, AMSTERDAM., NL ISSN: 0040-4039
- GANDOLFI, CARMELO ET AL: "(20.beta.H, 22.alpha.O,25R)-5.alpha.-spirostan-3.beta. -amino- 6.alpha.-ol and its isomers" TETRAHEDRON LETT. (1970), (19), 1677-80 , XP002168417
- DATABASE CHEMABS [Online] CHEMICAL ABSTRACTS SERVICE, COLUMBUS, OHIO, US; IRISMETOV, M. P. ET AL: "Modified steroids. XVI. Study of the Leuckart reaction in a series of steroid compounds of solasodine and diosgenin" retrieved from STN Database accession no. 94:121791 XP002168422 & IZV. AKAD. NAUK KAZ. SSR, SER. KHIM. (1980), (5), 81-3 ,
- DENINNO M P ET AL: "The C Radiolabelled Synthesis of the Cholesterol Absorption Inhibitor CP-148,623. A Novel Method for the Incorporation of a C Label in Enones" TETRAHEDRON,NL,ELSEVIER SCIENCE PUBLISHERS, AMSTERDAM, vol. 53, no. 32, 11 August 1997 (1997-08-11), pages 11007-11020, XP004105977 ISSN: 0040-4020
- DATABASE CHEMABS [Online] CHEMICAL ABSTRACTS SERVICE, COLUMBUS, OHIO, US; COLL, P. F. ET AL: "Steroidal compounds of Solanum antillarum O. E. Schulz. II" retrieved from STN Database accession no. 121:5053 XP002168423 & REV. CUBANA QUIM. (1992), 6(2), 66-71 ,
- CHEMICAL ABSTRACTS, vol. 54, no. 22, 25 November 1960 (1960-11-25) Columbus, Ohio, US; abstract no. 24855i, T. YAMAUCHI: "Saponins of Dioscareaceae. IX. Hydrolysis of Diosgenin glycosides" XP002168418 & CHEMICAL AND PHARMACEUTICAL BULLETIN, vol. 7, 1959, pages 343-348, TOKYO JP
- CHEMICAL ABSTRACTS, vol. 51, no. 16, 25 August 1957 (1957-08-25) Columbus, Ohio, US; abstract no. 12113d, S. G. BROOKS ET AL: "Synthesis of cortisone. XIX. Paper chromatography of some steroidal 11,12-diols and -ketols" XP002168419 & JOURNAL OF THE CHEMICAL SOCIETY, SECTION C: ORGANIC CHEMISTRY., 1957, pages 1175-1185, CHEMICAL SOCIETY. LETCHWORTH., GB ISSN: 1472-7781

## Description

The present invention relates to substituted sapogenins and their medicinal use, in particular in treating cognitive disfunction and allied conditions; and to compositions for use in such treatments. The invention is also concerned with the use of the compounds and compositions of the invention in regulating cellular activity, and with the treatment of conditions that are characterised by a deficiency in the number or function of membrane-bound receptors. In the following, the present invention will be described principally with reference to the treatment of Alzheimer's disease (AD) and senile dementia of the Alzheimer's type (SDAT), where deficiencies in a number of receptor types have been demonstrated. However, it is to be understood that the present invention relates generally to the treatment of conditions attributable to intrinsic pathological conditions and/or exposure to adverse environmental conditions these conditions being characterised by a deficiency in the number or function of membrane-bound receptors or a deficiency in transmission at the junctions between neurones or at the junctions of neurones and effector cells.

Conditions of the type mentioned above include Parkinson's disease, Lewi body dementia, postural hypotension, autism, chronic fatigue syndrome, Myasthenia Gravis, Lambert Eaton disease, diseases and problems associated with Gulf War Syndrome, occupational exposure to organophosphorus compounds and problems associated with ageing.

Alzheimer's disease (AD) and senile dementia of the Alzheimer's type (SDAT) are grave and growing problems in all societies where, because of an increase in life expectancy and control of adventitious disease, the demographic profile is increasingly extending towards a more aged population. Agents which can treat, or help in the management of, AD/SDAT are urgently required.

Age-associated memory impairment (AAMI) is a characteristic of older patients who, while being psychologically and physically normal, complain of memory loss. It is a poorly defined syndrome, but agents which are effective in treatment of AD/SDAT may also be of value in these patients.

Research into AD/SDAT is being carried out by traditional and conventional medical research methods and disciplines. In conventional medicine, there are several approaches to the treatment of AD/SDAT. It is known that the biochemical processes subserving memory in the cerebral cortex are (at least in part) cholinergically-mediated. Those skilled in the art will know that "cholinergically mediated" mechanisms may be directly attributable to acetylcholine acting on receptors, and these are direct effects. Other, clinically useful effects may also be caused by modulation of release of acetylcholine from pre-synaptic nerve endings or inhibition of enzymes that destroy acetylcholine. These modulating factors may be exerted through neurones where the mediator is non-cholinergic; these are referred to as indirect effects. Some attempts at treatment have focussed on the role of other mediators such as 5-hydroxytryptamine, which is a mediator in other areas of brain, such as the mid-brain nuclei. However, since fibres from these areas are projected forward into the cerebral cortex where the primary transmitter is acetylcholine, attention has focussed on the management of this mediator in the search for appropriate therapeutic agents.

Cholinergic strategies for the treatment of AD/SDAT have been directed at several points along the pathway of formation, synaptic release and removal of released acetylcholine.

One approach involves treatment with high doses of lecithin and other precursors of acetylcholine. This is of limited use in producing sustained improvements in cognitive performance.

Another approach involves the use of vegetable drugs such as Polygalae root extract, which has been shown to enhance choline-acetylcholine transferase (CAT) activity and nerve growth factor (NGF) secretion in brain. Oral administration of NGF has no effect on central nervous system neurons because it is a high molecular weight protein that cannot pass through the blood-brain barrier. However, agents which can pass through the blood-brain barrier and have a stimulating effect on NGF synthesis in the central nervous system have been proposed for the improvement of memory-related behaviour.

The results of a third clinical approach, which uses cholinesterase inhibitors such as tacrine hydrochloride, have been marginally more positive than the above. Substances obtained from plants used in Chinese and Western medicine, for example huperzine, galanthamine, and physostigmine have all been shown to be of some - although limited - benefit in the treatment of AD/SDAT in clinical studies and also in laboratory models. All of these substances are inhibitors of acetylcholine esterase (AChE). In patients with AD/SDAT, there may be reduced synthesis of acetylcholine (ACh), reduced efficiency in release of ACh from presynaptic stores, and a decrease in the number or function of postsynaptic (M₁) receptors. Reductions in pre-synaptic M₂ receptors have also been shown. The beneficial effect of AChE inhibitors is attributed to enhancement of acetylcholine levels at synapses in brain by slowing down the destruction of released transmitter.

Compositions which modulate cholinergic function are known to affect memory and recall. For example, nicotine stimulates nicotinic acetylcholine receptors, and the short lived memory enhancing effects of cigarette smoking are thought to be due to the effect of nicotine. Scopolamine, an antagonist of acetylcholine, will produce amnesia and impaired cognitive function manifesting in psychomotor tests as a prolongation of simple reaction times, possibly as a result of impaired attention, and is used for this purpose as an adjunctive analgesic treatment. The amnesic effect of scopolamine can be antagonised by nicotine.

There are two families of nicotinic receptor subtypes (α and β), and each includes four subgroups which differ in ligand specificity. The role of nicotinic receptors in the CNS is not well understood at the molecular level. It is possible that agents binding to nicotinic receptors may modify the rate of turnover at muscarinic receptor sites in brain. Nicotinic receptors are ligand-gated ion channels, and their activation causes a rapid (millisecond) increase in cellular permeability to Na⁺ and Ca⁺⁺, depolarisation and excitation.

Another class of cholinergic receptors can be stimulated by muscarine. Such muscarinic (M) receptors are G protein-coupled receptors. Responses of muscarinic receptors are slower; they may be excitatory or inhibitory. They are not necessarily linked to changes in ion permeability. Five types of muscarinic receptors have been detected by cholinergic receptor cloning, and are designated as m₁-m₅. Pharmacological effects are associated with four of the cloned receptors and they are designated as M₁-M₄ based on pharmacological specificity.

Using specific receptor proteins and monoclonal antibodies, it has been possible to further localise muscarinic receptors in brain as m₁ (postsynaptic) and m₂ (presynaptic). In heart, M₂ receptors are postsynaptic. Presynaptic muscarinic receptors are thought to be inhibitory, the binding of ACh to these receptors attenuating the release of further ACh to provide a negative feedback mechanism for Ach release. Selective M₂ receptor antagonists which are preferentially distributed to the brain may therefore be useful in treating Alzheimer's disease.

It is known that, in disease states such as AD/SDAT, there is general neuronal loss and deficits in cholinergic nerve function. It has been speculated that the high affinity nicotinic binding sites in the remaining cholinergic neurons might be converted to low affinity binding sites in treating such diseases, thereby sustaining transmitter release. By lowering the affinity of the nicotinic binding sites, a quick desensitising process is avoided.

Agonist activation at nicotinic receptors in brain has rapid onset and offset. A decreased affinity of the nicotinic receptors will reduce the desensitisation process. Schwarz RD. et al (J. Neuro Chem 42, (1984), 1495-8) have shown that nicotine binding sites are presynaptically located on cholinergic (and also 5-hydroxytryptaminergic and catecholaminergic) axon terminals. A change in high affinity binding sites on AD/SDAT may also induce a change in the modulatory effect the nicotinic binding sites may have on other transmitter systems.

Presynaptic cholinergic mechanisms are also under inhibitory control by GABAergic neurons and this inhibition is thought to be intensified in AD/SDAT. Removal or reduction of this inhibition intensifies presynaptic cortical cholinergic activity and enhances cognitive processing.

The interactions of interneuronal fibres innervated by nicotine (reducing binding affinity), and dis-inhibition of GABAergic fibres both have a presynaptic locus.

This is a simplistic model of central transmission, but provides a framework for understanding the attempts which have been made to increase the effective concentration of acetylcholine in central synapses. This further illustrates the concept of direct and indirect action. There are disadvantages attaching to the three conventional therapeutic approaches to AD/SDAT treatment mentioned above: ACh precursor supplementation, agonist replacement and acetylcholine esterase inhibition. These treatments may result in a short-term increase in the availability of ACh which may activate feedback mechanisms resulting in the desensitisation of postsynaptic receptors. On theoretical grounds, long term benefits would not be predicted and when treatment is interrupted, any benefits in management of AD/SDAT and AAMI disappear and the condition may even be aggravated.

It has been shown that a compound with M₁ agonist and M₂/M₃ antagonist activity improved cognitive performance in SDAT patients (Sramak et al, Life Sciences voL 2, No. 3,195-202, 1997). However, this compound causes unacceptable cholinergic side effects, such as fatigue, diarrhoea and nausea.

A more radical approach to AD/SDAT and AAMI aims to increase the number of postsynaptic (M₁) receptors, in brain. It is known from Chinese Patent No. GN1096031A, that sarsasapogenin (SaG) can up-regulate M₁ cholinergic receptors and also down-regulate (i.e, move towards normal levels of) β-adrenergic receptors, the number of which may be pathologically-raised in AD/SDAT.

Patent applications have been published which claim the usefulness of a number of steroid sapogenins having spirostane, furo-spirostane, spirosolane or solanidine structures in the treatment of diseases.

The following patent publications are of particular relevance here: Chinese Patent Application No. CN 1096031A discloses two way regulatory effects of the spirostane sapogenin, sarsasapogenin, on β-adrenergic and M-cholinergic receptors. The disclosure in this document, however, is brief The other document of relevance is patent publication DE 4303214A1 which claims the use of a very wide range of saponins and sapogenins in the treatment of a whole range of diseases that the inventors consider to be of viral origin. This disclosure is however of dubious value in that it is well recognised that there is no infective element to a very large number of the conditions that are characterised by deficient synaptic transmission and thus the basic premise of the alleged invention is flawed In addition they present no data of any kind that allows one skilled in the art to be able select a preferred compound from the large number that are claimed. Finally, the International patent application WO 99/48547 discloses the activity of unsubstituted sapogenins such as sarsasapogenin, smilagenin, prazerigenin, astragaloside, tigogenin, hecogenin, ruscogenin and diosgenin on M2 receptors and their use for treating Alzheimer's disease.

The inventors have now found that substituted sapogenin derivatives exhibit enhanced ability to regulate receptors and/or to increase the number of M2 receptors in the brain, in comparison of the compounds of the prior art. Moreover, they show unexpected good bioavailability over the known compounds and are thus particularly suitable active ingredients for treating M2 receptors related diseases such as the Alzheimer's disease.

Thus, an object of the present invention is the provision of substituted sapogenin derivatives.

According to another aspect of the invention, there is provided the use of a substituted sapogenin derivative in the manufacture of a medicament for use in the regulation of cellular activity or for the treatment of a condition characterised by a deficiency in postsynaptic membrane-bound receptor number or function.

Those skilled in the art will be aware of the relationship between saponins and their sapogenins, and that the latter tend to be fat-soluble whereas the saponins tend to be water-soluble. Sapogenins are therefore better able to cross the blood-brain barrier. The skilled man will also be aware of the epimerisation of certain sapogenins under conditions of acid hydrolysis.

The variation in pharmacological properties and pharmacodynamic actions of various types of sapogenins underlines the need for selection ofthose agents which are most useful in the treatment of AD/SDAT. The discovery of novel facts about the action of sapogenin derivatives has made it possible to determine which substances are most useful for the treatment for the treatment of AD/SDAT and the like.

The term "substituted sapogenins" according to the invention refers to sapogenin derivatives as defined in and by and the appended claims bearing at least one substituent "X", wherein the X radical is chosen from the group consisting of :
- halo atom,
- (Me-S-), (Me-SO-), (Me-SO₂-),
- N₃-, NH₂-, MeSO₂NH-, and
- alky.

According to a preferred aspect, it refers to sapogenin derivatives wherein at least one OH-group is substituted by a X radical atom as defined above.

As used hereabove and hereafter, the term "sapogenin" derivatives includes compounds such as those described in the British applications GB 9923076.5, GB 9923077.3, GB 9923078.1, and such as those disclosed in the International application WO 99/49507, incorporated herein by reference, including sarsasapogenic, smilagenin, anzurogenin-D, hecogenin, sisalgenin, tigogenin, diosgenin, ruscogenin, prazerigenin and astragaloside.

According to a first aspect, suitable substituted sapogenins for treating cognitive dysfunction may be chosen from those of general formula (I): 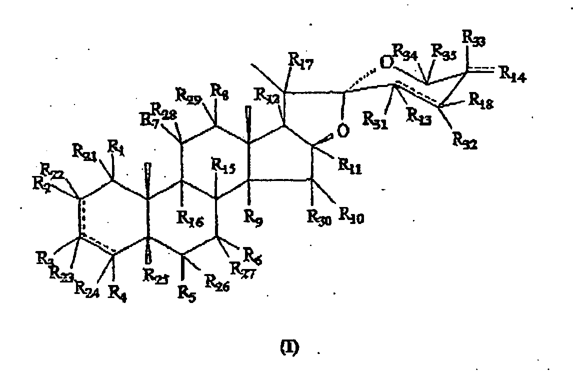 and pharmaceutically acceptable salts,
wherein in the general formula (I):
-R₁, R₂, R₄, R₅, R₆, R₇, R₈, R₁₀, R₁₃, R₂₀, R₂₁, R₂₂, R₂₃, R₂₄, R₂₆, R₂₇, R₂₈, R₂₉, R₃₀, R₃₁, R₃₂, R₃₄, R₃₅, are, independently of each other, either H, OH, =O, and OR where R = alkyl or acyl group or absent,
-R₉, R₁₁, R₁₂, R₁₅, R₁₆, R₁₇, R₂₅ are either a H. OH, OR where R = alkyl or acyl group or absent;
- R₃₃, R₁₄ =H; alkyl group, OH, = O or OR where R= alkyl or acyl group or absent.
represents an optional double bond, and
   wherein in addition to the above is a X radical,
wherein X is chosen from the group consisting of:
halo stom,
-(Me-S-), (Me-SO-), (Me-SO₂-),
N₃-, NH₂-, MeSO₂NH-, and
alkyl.

Preferably, in the general formula (I):
- R₁, R₂, R₄, R₅, R₆, R₇, R₈, R₁₀, R₁₃, R₂₀, R₂₁, R₂₂, R₂₃, R₂₄, R₂₆, R₂₇, R₂₈, R₂₉ R₃₀, R₃₁, R₃₂, R₃₄, R₃₅, are, independently of each other, either H. OH, =O, and OR where R= alkyl or acyl group or absent,
-R₉, R₁₂, R₁₅, R₁₅, R₁₇, =H,
-R₂₄, R₂₅, are either a H, OH, OR where R = alkyl or acyl group or absent;
- R₃₃, R₁₄ = H, alkyl group, OH, =O or OR where R = alkyl or acyl group or absent,
----- represents an optional double bond, and
wherein in addition to the above
R₃ is a X radical,
wherein X is chosen from the group consisting of:
• halo atom,
•(Me-S-), (Me-SO-), (Me-SO₂-).
-N₃-, NH₂-, MeSO₂NH-, and
alkyl

More preferably, in the general formula (I):
-R₁=R₂=R₄=R₅=R₆=R₇=R₈=R₁₀=R₁₁=R₉=R₁₂=R₁₃=R₁₅=R₁₆=R₁₇=R₁₈= -R₂₁=R₂₂=R₂₄=R₂₅=R₂₆=R₂₇=R₂₈=R₂₉=R₃₀=R₃₁=R₃₂=R₃₃=R₃₄=R₃₅=H,
-R₁₄=CH₃,
----- represents a single bond,
- the methyl group at C25 is either in the R or S configuration and R₃ is a X radical, R₂₃ being H, OH, = O, and OR where R = alkyl or acyl group or absent, and X is chosen from the group consisting of:
-halo atom,
-(Me-S-), (Me-SO-), (Me-SO₂-), and
-N₃-, NH₂-, MeSO₂NH-, and
-alkyl

Still more preferably, in the general formula (I):
-R₁=R₂=R₄=R₅=R₆=R₇=R₈=R₁₀=R₁₁=R₉=R₁₂=R₁₃=R₁₅=R₁₆=R₁₇=R₁₈= R₂₁=R₂₂=R₂₄=R₂₅=R₂₆=R₂₇=R₂₈=R₂₉=R₃₀=R₃₁=R₃₂=R₃₄=R₃₅=H,
-R₁₄ = R₃₃ = alkyl, e.g. methyl.
----- represents a single bond,
R₃ is a X radical, R₂₃ being H,
OH, =O, and OR where R = alkyl or acyl group or absent
and X is chosen from the group consisting of:
-halo atom,
-(Me-S-), (Me-SO-), (Me-SO₂-), and
-N₃-, NH₂-, MeSO₂NH-, and
-alkyl

According to a second aspect of the invention, suitable substituted sapogenins for treating cognitive dysfuntion may be chosen from the compounds of general formula (II): 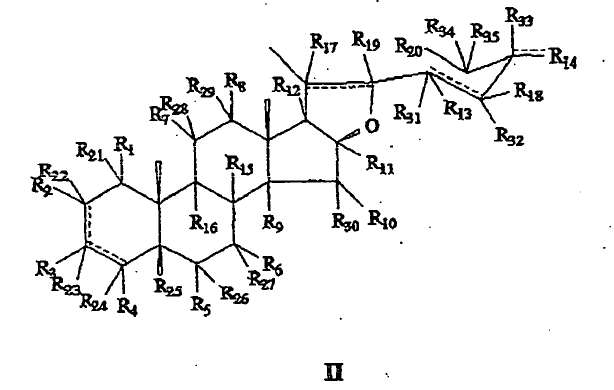 , their pharmaceutically acceptable salts.
wherein in the general formula (II):
-R₁, R₂, R₄, R₅, R₆, R₇, R₈, R₁₀, R₁₁, R₉, R₁₂, R₁₃, R₁₉, R₂₀, R₂₁, R₂₂, R₂₃, R₂₄, R₂₅, R₂₇, R₂₈, R₂₉, R₃₀, R₃₁, R₃₂ R₃₄, R₃₅, are, independently of each other, either H, OH, =O, and OR where R= alkyl or acyl group or absent;
- R₉, R₁₁, R₁₂, R₁₅, R₁₆, R₁₇, R₁₉, R₂₅ are either a H, OH, OR where R = alkyl or acyl
group or absent;
-R₃₃, R₁₄ =H, alkyl group, OH, =O or OR where R = alkyl or acyl group or absent,
----- represents an optional double bond, and
wherein in addition to the above
is a X radical,
wherein X is chosen from the group consisting of:
-halo atom,
-(Me-S-), (Me-SO-), (Me-SO₂-),
·N₃-,NH₂-,MeSO₂NH-, and
- alkyl.

Preferably, in the general formula (II)
- R₁, R₂, R₄, R₅, R₆, R₇, R₈, R₁₀, R₁₃, R₁₈, R₂₁, R₂₂, R₂₃, R₂₄, R₂₆, R₂₇, R₂₈, R₂₉ R₃₀, R₃₁, R₃₂, R₃₄, R₃₅, are, independently of each other, either H, OH, =O, and OR where R = alkyl or acyl group or absent;
-R₉, R₁₂, R₁₅, R₁₆, R₁₇ = H,
-R₂₉ = either H, OH, =O, and OR where R = alkyl, acyl or carbohydrate and
-R₁₂, R₁₉, R₂₅, are either a H, OH, OR where R= alkyl or acyl group or absent;
-R₃₃ R₁₄, =H, alkyl, group, OH, =O or OR where R = alkyl or acyl group or absent,
----- represents an optional double bond, and
wherein in addition to the above
R_{3,}
is a X radical,
wherein X is chosen from the group consisting of:
· halo atom,
· (Me-S-), (Me-SO-), (Me-SO₂-),
· N₃-, NH₂-, MeSO₂NH-, and
· alkyl.

More prefebly, in the general formula (II):
-R₁=R₂=R₄=R₅=R₆=R₇=R₈=R₁₀=R₁₁=R₉=R₁₂=R₁₃=R₁₅=R₁₆=R₁₇₌R₁₈= -R₂₁=R₂₂=R₂₃=R₂₄=R₂₅=R₂₆=R₂₇=R₂₈=R₂₉=R₃₀=R₃₁=R₃₂=R₃₃=R₃₄=R₃₅=H,
-R₁₄=CH 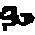 ,
-R₂₀ = OH or -OR, where, R = alkyl, acyl or carbohydrate and R₁₉ = H or is absent
----- represents an optional double bond, and
-the methyl group at C25 is either in the R or S configuration and
wherein in addition to the above
R₃ is a X radical, R₂₃ being H, OH, =O, and OR where R= alkyl of acyl group or absent,
and X is chosen from the group consisting of:
- halo atom,
- (Me-S-), (Me-SO-), (Me-SO₂-), and
-N₃-, NH₂, Me-SO₂NH-, and
-alkyl

Still more preferably, in the general formula (II):
-R₁=R₂=R₄=R₅=R₆=R₇=R₈=R₁₀=R₁₁=R₉=R₁₂=R₁₃=R₁₅=R₁₆=R₁₇=R₁₈= -R₂₁=R₂₂=R₂₃=R₂₄=R₂₅=R₂₆=R₂₇=R₂₈=R₂₉=R₃₀=R₃₁=R₃₂=R₃₄=R₃₅=H,
-R₁₄=R₃₃=alkyl, e.g. methyl,
- R₂₀=-OH or -OR where R = alkyl, acyl or carbohydrate and R₂₉ = H or is absent
----- represents an optional double bond, and
wherein in addition to the above
R₃ is a X radical, R₂₃ being H,
OH, =O, and OR where R = alkyl or acyl group or absent,
and X is chosen from the group consisting of:
^{.} halo atom,
^{.} (Me-S-), (Me-SO-), (Me-SO₂-), and
^{.} N₃-, NH₂-, MeSO₂NH-, and
^{.} alkyl.

According to a third aspect, suitable substituted sapogenins for treating cognitive dysfunction may be chosen from the compounds of general formula (III): 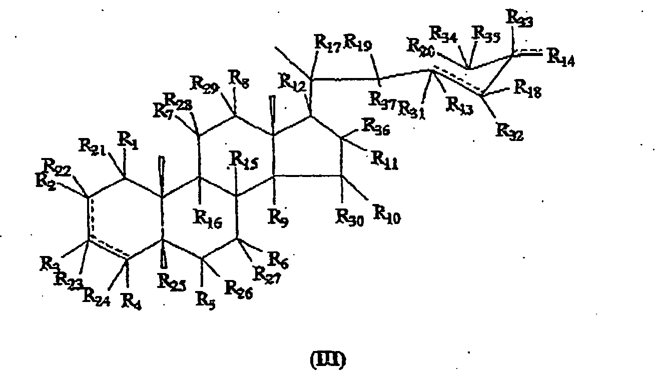 and their pharmaceutically acceptable salts,
wherein the general formula (III):
-R₁, R₂, R₄, R₅, R₆, R₇, R₈, R₉, R₁₀, R₁₃, R₁₈, R₁₉, R₂₀, R₂₁, R₂₂, R₂₃, R₂₄, R₂₆, R₂₇, R₂₈, R₂₉, R₃₀, R₃₁, R₃₂, R₃₄, R₃₅, R₃₆, R₃₇ are, independently of each other, either H, OH,
=O, and OR where R = alkyl or acyl group or absent,
- R₉, R₁₁, R₁₂, R₁₄, R₁₅, R₁₆, R₁₇, R₂₅, R₃₃ can be either a H, OH, OR where R = alkyl or acyl group or absent;
- R₁₄ = R₃₃ = alkyl = H alkyl group, OH,= O or OR where R = alkyl or acyl group or absent,
represents an optional double bond, and
wherein in addition to the above
is a X radical,
wherein X is chosen from the group consisting of:
halo atom,
-(Me-S-), (Me-SO-), (Me-SO₂-),
N₃-, NH₂-, MeSO₂NH-, and
alkyl.

Preferably, in the general formula (III):
- R_{1,}R₂, R_{4,} R₅, R₅, R₆, R₇, R₈, R₁₀, R₁₃, R₁₈, R₁₉, R₂₁, R₂₂, R₂₃, R_{24,} R₂₅, R₂₇, R₂₈, R₂₉, R₃₀, R₃₁, R₃₂, P₃₄, R₃₅, R₃₆, R₃₇ are, independently of each other, either H, OH, =O. and OR where R= alkyl or acyl group or absent;
- R₉, R₁₂, R₁₅, R₁₆, R₁₇=H,
-R₂₀=H, OH, =O, and OR where R= alkyl, acyl or carbohydrate and
- R₁₁, R₂₅, are either a H, OH, OR where R = alkyl or acyl group or absent;
- R₃₃, R₁₄ = H, alkyl group, OH, =O or OR where R = alkyl or acyl group or absent,
represents an optional double bond, and
wherein in addition to the above
is a X radical,
wherein X is chosen from the group consisting of:
halo atom,
-(Me-S-), (M[e-SO-),(Me-SO₂-),
N₃-, NH₂-, MESO₂NH-, and
alkyl.

More preferably, in the general formula (III):
-R₁=R₂=R₄=R₅=R₆=R₇=R₈=R₁₀=R₁₁=R₉=R₁₂=R₁₃=R₁₆=R₁₇=R₁₈= R₂₁=R₂₂=R₂₃=R₂₄=R₂₅=R₂₆=R₂₇=R₂₈=R₂₉=R₃₀=R₃₁=R₃₂=R₃₃=R₃₄=R₃₅=H,
-R₁₄ =CH₉,
- R₂₀=OH or -OR where R = alkyl, acyl or carbohydrate and
R₁₉ = H or is absent
R₃₇ = H,-OH or =O R₃₆= H a or -OH
----- represents a single bond, and
- the methyl group at C25 may be either in the R or S configuration and wherein in addition to the above
R₃ is a X radical, R₂₃ being H,
OH, =O, and OR where R= alkyl or acyl group or absent,
and X is chosen from the group consisting of:
halo atom,
- (Me-S-), (Me-SO-), (Me-SO₂-), and
-N₃-, NH₂-, MeSO₂NH-,
alkyl.

Still more preferably, in the general formula (III):
-R₁= R₂= R₄= R₅= R₆=R₇=R₈=R₁₀=R₁₁=R₉=R₁₂=R₁₃=R₁₅=R₁₆= R₁₇=R₁₈= R₂₁=R₂₂=R₂₃=R₂₄=R₂₅=R₂₆=R₂₇=R₂₈=R₂₉=R₃₀=R₃₁=R₃₂=R₃₄=R₃₅=H
-R₁₄=R₃₃=alkyl, e.g. methyl,
- R₂₀= -OH or -OR where R = alkyl, acyl or carbohydrate and
R₁₉ = H or is absent
R₃₇= H, -OH or =O
R₃₆= H or -OH
----- represents a single bound, and
wherein in addition to the above
R₃ is a X radical, R₂₃ being H, OH, =O, and OR where R= alkyl or acyl group ot absent,
and X is chosen from the group consisting of :
- halo atom,
- (Me-S-), (Me-SO-), (Me-SO₂-), and
-N₃-,NH₂-,MeSO₂NH-,
-alkyl.

According to a preferred aspect, compounds for use in the invention may be chosen from:
(3β-fluoro-5β,20α,22α,25R-spirostane), (3,3-difluoro-5β, 20α,22α,25R-spirostane), (3α-methylsulphonylamino-5β,20α,22α,25R-spirostane, (3α-azido-5β,20α,22α,25R-spirostane) and (3α-amino-5β,20α,22α,25R-spirostane).

Preferentially, compounds for use in the invention may be chosen from sarsasapogenin, episarsasapogenin, smilagenin and epismilagenin, see each case substituted at the R₃ site by X,
wherein X is chosen from the group consisting of :
• halo atom,
• (Me-S-), (Me-SO-), (Me-SO₂-),
• N₃-, NH₂-, MeSO₂NH-, and
• alkyl.

In the formulae given here where the possible substituents are defined in groups (e.g. as with R₁₄ and R₃₃) the substituents may be the same or different. As used hereabove and hereafter
"Halo" means fluoro, chloro, bromo, or iodo. Preferred are fluoro, chloro or bromo, and more preferred is fluoro.

"acyl" means a H-CO- or Alkyl-CO- group wherein the alkyl group is as herein described. Preferred acyls contain a lower alkyl. Exemplary acryl groups include formyl, acetyl, propanoyl, 2-methylpropanoyl, butanoyl and palmitoyl.

"-O-acyl" means a H-CO-O- or Alkyl-CO-O- group wherein the alkyl group is as herein described.

- "Alkyl" means an aliphatic hydrocarbon group which may be straight or branched having 1 to 20 carbon atoms in the chain. Preferred alkyl groups have 1 to about 12 carbon atoms in the chain. Branched means that one or more lower alkyl groups such as methyl, ethyl or propyl are attached to a linear alkyl chain. "Lower alkyl" means about 1 to about 4 carbon atoms in the chain which may be straight or branched. Exemplary alkyl groups include methyl, ethyl, *n*-propyl, *i-*propyl, *n*-butyl, *t*-butyl, *n*-pentyl, 3-pentyl.

The term "pharmaceutical composition" means a composition comprising a compound of formula I or II and at least one component selected from the group comprising pharmaceutically acceptable carriers, diluents, adjuvants, excipients, or vehicles, such as preserving agents, fillers, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms.

"Pharmaceutically acceptable" means it is, within the scope of sound medical judgement, suitable for use in contact with the cells of humans and lower animals without undue toxicity, irritation, allergic response and the like, and are commensurate with a reasonable benefit/risk ratio.

"Pharmaceutically acceptable dosage forms" means dosage forms of the compound of the invention, and includes, for example, tablets, dragees, powders, elixirs, syrups, liquid preparations, including suspensions, sprays, inhalants tablets, lozenges, emulsions, solutions, granules, capsules and suppositories, as well as liquid preparations for injections, including liposome preparations. Techniques and formulations generally may be found in Remington, Pharmaceutical Sciences, Mack Publishing Co., Easton, PA, latest edition.

"Pharmaceutically acceptable salts" means the relatively non-toxic, inorganic and organic acid addition salts, and base addition salts, of compounds of the present invention. These salts can be prepared *in situ* during the final isolation and purification of the compounds. In particular, acid addition salts can be prepared by separately reacting the purified compound in its free base form with a suitable organic or inorganic acid and isolating the salt thus formed. See, for example S.M. Berge, et al., Pharmaceutical Salts, J. Pharm. Sci., 66: p.1-19 (1977). Base addition salts can also be prepared by separately reacting the purified compound in its acid form with a suitable organic or inorganic base and isolating the salt thus formed. Base addition salts include pharmaceutically acceptable metal and amine salts.

### Preparation of Compounds of the Invention

According to a further aspect of the invention, there is provided a process of preparation of the compounds of the invention.

The starting materials for the preparation of compounds of the inventions as hereinbefore defined, can be hydroxylated spirostanes, including (but not limited thereto) smilagenin, sarsasapogenin, and their stereoisomers, such as epismilagenin and episarsasapogenin. Smilagenin, sarsasapogenin, epismilagenin are commercially available. Suppliers are well-known from the one skilled in the art and may include Sigma Aldrich, Research Plus Inc., Steraloids Inc., etc...
Episarsasapogenin is available as a starting material by literature methods (Thompson et al JACS 5225 (1959).
Also, as starting products, unsubstituted sapogenins may occur naturally in a range of plant species, notably from the genera Smilax, Asparagus, Anemarrhena, Yucca and Agave. The species presently of greatest interest include Smilax regelii Kilip & Morton - commonly known as Honduran sarsaparilla; Smilax aristolochiaefolia Miller - commonly known as Mexican sarsaparilla; Smilax ornata Hooker - commonly known as Jamaican sarsaparilla; Smilax aspera - commonly known as Spanish sarsaparilla; Smilax glabra Roxburgh; Smilax febrifuga - Kunth -commonly known as Ecuadorian or Peruvian sarsaparilla; Anemarrhena asphodeloides Bunge; Yucca schidigera Roezl ex Ortgies; and Yucca brevifolia Engelm.
Unsubstituted sapogenins may also occur naturally in other genera, for example Dioscorea, Trillium, Solanum, Strophanthus, Digitalis and Trigonella. However, some sapogenin derivatives from these sources possess undesirable properties and are thus not recommended for use in the invention.

According to a first aspect, substituted compounds of formula (I), wherein X and R₁, R₂, R₃ etc are as hereinbefore defined, may be prepared from the corresponding hydroxlyated compound wherein the hydroxyl group has been substituted by X as hereinbefore defined.

The hydroxyl group is activated by conversion to good leaving group such as a tosylate, mesylate, triflate or halide (often in the presence of basic aromatic amines), followed by displacement of this leaving group with either an azide, methylsulphyl, alkyl or fluoro nucleophile (Scheme 1).
This reaction is preferably carried out in the presence of protic solvents.

Conversion of the resultant azido compounds (when the nucleophile is azido) to amines is accomplished via a reductive step. The resulting amino group could be further reacted with methanesulphonyl chloride to form the corresponding methanesulphonylamino compounds. Conversion of the methylsulphyl compounds (formed when the reaction of the leaving group above is with the methylsulphyl nucleophile) to the methylsulphinyl and methylsulphonyl compounds is accomplished by oxidative steps.

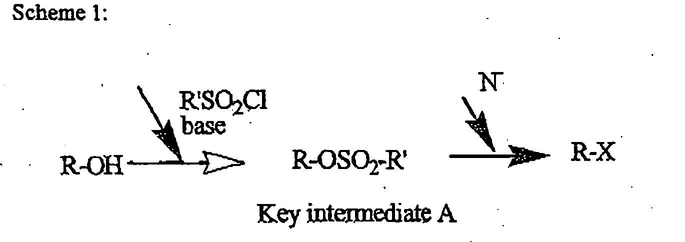

The compounds thus prepared may be recovered from the reaction mixture by conventional work-up/extraction methods followed by purification by chromatography and recrystallisation techniques

Key intermediates of formula A, may be prepared by reaction of the corresponding hydroxylated compounds, for example smilagenin, with sulphonyl chlorides (for example methanesulphonyl chloride) or sulphonyl anhydrides (for example trifluoromethanesulphonyl anhydride), which are commercially available, in the presence of base (typically pyridine or derivatives thereof).

Two representative examples of the preparation of intermediates A of the invention are specifically described in the experimental part (see Preparation Examples 1-2).

Two representative examples of the nucleophilic attack on key intermediates A are described in Examples 1-2.

A representative example of the reduction of the azide group formed by nucleophilic attack on key intermediate A is described in Example 3.

A representative example of the formation of methanesulphonylamino compounds from the corresponding amine is described in Example 4.

Alternatively, according to a second aspect, substituted compounds of formula (I), wherein X and R₁, R₂ R₃ etc are as hereinbefore defined, may be prepared from may also be prepared from the corresponding keto compounds. The corresponding keto compounds are themselves prepared by oxidation of the corresponding hydroxylated compound. Nucleophilic attack on the sp₂ centre of the carbonyl with a variety of nucleophiles including alkyl results in the X group being introduced along with a tertiary alcohol which may eliminate to afford a double bond (Scheme 2).

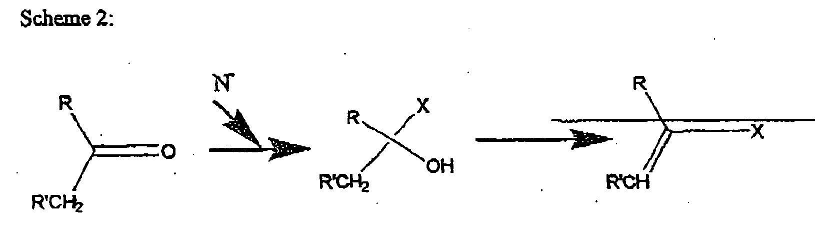

Compounds of formula (I), wherein X, R₁, R₂, R₃ etc are as hereinbefore defined, where there are two fluoro atom attached at one carbon centre can be formed by reaction of the corresponding keto compounds with SF4, SeF4 or more commonly dialkylaminosulpur trifluoride compounds (eg (dimethylamino) sulphur trifluoride, DAST).

A representative example of the preparation of one such compound is described in the experimental part (see Example 5).

Methods for the introduction of alkyl substitutents in addition to those mentioned above include the reaction of copper lithium alkyl reagents with α,β unsaturated ketones via a Michael type 1,4 addition.
A representative example of the general methodology for the introduction of a C25 methyl group to smilagenin (or its derivatives such as sarsasapogenin) is described in Comparative Example 1.

According to a further aspect, compounds of the invention may be prepared by interconversion of other compounds of the invention.
Examples of the conversion of compounds of formula (I), wherein X, R₁, R₂, R₃
etc are as hereinbefore defined, to the corresponding compounds of formula (II) and formula (III) have been reported by others (for example Marker et al JACS, 846 (1939), Wall et al JACS, 340 (1955), Wall et al JACS 3086 (1955)).

In general terms, steps of the process of the invention may be carried out by application or adaptation of known methods, by which is meant methods used heretofore or described in the literature, for example those described by R.C. Larock in Comprehensive Organic Transformations, VCH publishers, 1989.

In the reactions described hereinafter it may be necessary to protect reactive functional groups, for example hydroxy or carboxy groups, where these are desired in the final product, to avoid their unwanted participation in the reactions. Conventional protecting groups may be used in accordance with standard practice, for examples see T.W. Green and P.G.M.Wuts in "Protective Groups in Organic Chemistry" John Wiley and Sons, 1991; J. F. W. McOmie in "Protective Groups in Organic Chemistry" Plenum Press, 1973.

The compound thus prepared may be recovered from the reaction mixture by conventional means. For example, the compounds may be recovered by distilling off the solvent from the reaction mixture or, if necessary after distilling off the solvent from the reaction mixture, pouring the residue into water followed by extraction with a water-immiscible organic solvent and distilling off the solvent from the extract. Additionally, the product can, if desired, be further purified by various well techniques, such as recrystallization, reprecipitation or the various chromatographs techniques, notably column chromatography or preparative thin layer chromatography.

The present invention enables a pharmaceutical composition having cognitive function enhancing properties which comprises an effective amount of a substituted sapogenin of the invention.

The substituted sapogenins used in the present invention are steroidal; they are preferably non-oestrogenic in effect.

The invention enables a pharmaceutical composition having cognitive function enhancing properties which comprises an effective amount of a subsbituted sapogenin of the invention in the form of a compound prepared from an extract derived from a plant of the genus Smilax, Asparagus, Anemarrhena, Dioscorea, Yucca or Agave.

The invention further enables the use of an extract of a plant of the genus Smilax, Asparagus, Anemarrhena. Dioscorea, Yucca or Agave in the preparation of the compounds for use in a pharmaceutical composition according to the invention.

The invention enables the use of the compositions defined above. Thus, according to a further aspect, the present invention provides a non-therapeutic method of enhancing cognitive function which comprises administering to a human or animal an effective dosage of a composition of the invention.

The invention also provides a non-therapeutic method of enhancing cognitive function in a human or non-human animal, which comprises administering an effective dose of substituted sapogenins of the invention.

As used herein, the term "cognitive function" refers to functions such as thinking, reasoning, remembering, imagining and learning.

According to a further aspect, the invention also enables compositions having cognitive function enhancing properties which comprises at least two, preferably two, substituted sapogenins of the invention.

According to a still further aspect, the invention also concerns the use of one or more of the said derivatives of the invention in the manufacture of a foodstuff or beverage to have cognitive function, enhancing effect when ingested. The said foodstuff or beverage comprising an effective quantity of one or more of the said derivatives of the invention to have cognitive function enhancing effect when ingested, is also part of the present invention.

In identifying compounds that would have use in the treatment of SDAT and other diseases characterised by reductions in receptor number or synaptic transmission, the inventors have given consideration to the need to identify compounds that would have the desired effect but would be devoid of any oestrogenic effects, as these would be unacceptable, particularly in male patients. A number of the compounds claimed to have activity in patent application DE 4303214A1 have marked oestrogenic activity and are therefore unacceptable. Preferably, substituted sapogenins of the present invention however, do not display oestrogenic activity. In addition these compounds were tested at other steroid receptors and were found to have no activity at any of the following receptors:
Progesterone
Glucocorticoid
Testosterone.

In order to illustrate the invention further by way of non-limiting example, reference will now be made to the accompanying drawing and to the Example which follows; in the drawings:

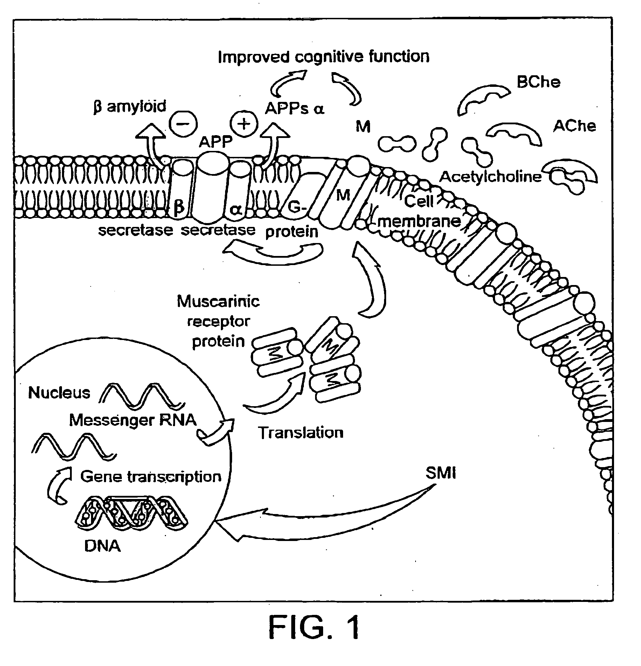

FIGURE 1 illustrates a hypothetical mode of action for sapogenin derivatives;

Referring to Fig. 1, a diagrammatic representation of the function of sapogenin derivatives of the invention is shown. It is believed that substituted sapogenins act primarily on cell nuclei; the invention is not, however, limited to any particular mode of action. The observed increase in muscarinic receptor number consequential upon administration of sapogenin derivatives is interpreted as leading to increased expression of muscarinic receptor protein. The possible link between the secretases and β-amyioid protein formation (discussed above) is indicated in the drawing.

### Preparation of the compounds of the invention

The following examples are provided to illustrate the process of preparation of the invention in a non-limiting manner.

### Preparation Example 1:3β-Methylsulfonyloxy-5β,20α,22α,25R-spirostan

Methanesulfonyl chloride (1.83g, 16.0 mmol) was added to a solution of smilagenin (5.0 g, 12.0 mmol) in dry pyridine (40ml). The mixture was heated on a steam bath for.10 min, allowed to stand overnight at room temperature and then poured onto ice-water (80 ml). Trituration. gave an off-white solid, which was removed by filtration and washed with water. This material was dried in a vacuum dessicator over CaCl₂ to give 5.70g of crude product (used as such in the reactions described bellow). A sample (1.0g) was reaystallised from acetone (2x) to give 340mg of the above mesylate as a white powder solid, mp 135-137 °C. m/z 494 (M⁺ for G₂₈H₄₆SO₅); R_{f} 0.4 (silica, ethyl acetate-hexane,1: 4)

### Preparation Example 2: 3α-trifluoromethanesulphonoxyl-5β,20α,22α,25R-spirostane

A solution of trifluoromethanesulphonic anhydride (0,41 ml) in anhydrous dichloromethane (DCM, 3.5 ml) was added dropwise over 30 min to a solution of epismilagenin (0.7 g, 1.68 mmol) in anhydrous DCM (10 ml) and anhydrous pyridine (0.24 ml)that had been cooled to 0°C. The reaction was stirred at 0°C for 1 h whereupon DCM (100 ml) was added. The solution was washed with saturated sodium bicarbonate solution (50 ml), dried (MgSO₄), filtered and evaporated in *vacuo* to afford 3α-trifluoromethanesulphonoxyl-5β,20α,22α,25R-spizostane as a yellow solid (2 g).

### Example 1: 3α-Azido-5β,20α,22α,25R-spirostau (3-azido-3-deoxy-epismilagenin)

3β-Methylsulfonyloxy-5β,20α,22α,25R-spirostan (see Preparation Example 1 - 1.0g, 2.02 mmol) was dissolved in DMPU (N,N'-dimethylpropyleneurea, 12 ml) and sodium azide (1.0 g, 15.4 mmol) was added. The mixture was stirred at 40-45°C for 48h then partitioned between water (50 ml) and ether (50 ml). The aqueous layer was extracted with ether (4x), the combined organic phases washed with water and dried over MgSO₄. The solvent was removed in vacuo to give an oily solid (10g). Trituration in ether-hexane and recrystallisation from acetone gave 620 mg of the above azide as white crystals,
mp 153-156°C.
m/z 441 (M⁺ for C₂₇H₄₃N₃O₂). v ₘₐₓ (nujul) 2091 can⁻¹. δ_{H} (270MHz, CDCl₃) 0.75 (3H, s, 18-CH₃), 0.80 (3H, d, *J*=-6.2 Hz, 27.-CH₃),0.95 (3H, s, 19-CH₃), 0.97 (3H, d, *J* = 6.9 Hz, 21-CH₃), 1.0-2.05 (27H, complex m, aliphatics), 3.2.3.5 (3H, complex m, H-26,
H-3), 4.39 (1H, br dd, *J* = 6.4, 8.5 Hz, H-16). δ_{C} (270 MHz, CDCl₃) 14.5, 16.4, 17.1, 20.6, 23.5, 26.6, 26.7, 27.0, 23.8, 30.3, 31.4, 31.3, 32.5, 34.8, 35.5, 35.6, 40.1, 40.6, 41.6, 42.3, 56.2 (C-14), 61-2 (C-17 or C-3), 62.3 (C-3 or C-17), 66.9 (C-26), 80.9 (C-16), 109.2 (C-22).
R_{f} 0.8 (silica, ethyl acetate-hexane, 1:19)

### Example 2: 3β-fluoro5β,20α,22α,25R-spirosytane

3α-trifluoromethanesulphonoxyl-5β,20α,22α,25R-spirostane (see Preparation Example2 - 2 g) was dissolved in dry DCM (11 ml) to which was added tetrabutylammonium fluoride (1 M in THF, 8,4 ml). The reaction was heated at reflux for 1.5 h, cooled, and diethyl ether (150 ml) added. The solution was washed with brine (2 x 30 ml), dried (MgSO₄), filtered and evaporated in vacuo to afford crude 3β-fluoro-5β,20α,22a,25R-spirostane (3 g) as an orange semi-solid material. Purification by column chromatography using DCM/petroleum ether (1:4) as eluant afforded the desired product (353 mg). Recrystallization from acetone afforded 3β-fluoro-5β,20α,22α,25R-spirostane as a white solid (92.7 mg) TLC: (Silica gel) 20%EtOAc/petrol Rf 0.57; melting point: 166-167°C; ¹H nmr spectrum (CDCl₃, 270 MHz): partial data 4.85 (1H d, J=48.Hz), 4.40 (1H,q), 3.50 (1H,dd) 3.40 (1H,t), 0.98 (3H,s), 0.95 (3H,d), 0.80 (3H, d), 0.75 (3H,s) ppm; ¹³C nmr spectrum (CDCl₃, 68 MHz): 109.26,90.00 (J= 250 Hz), 80.87, 66.85, 62.25, 56.46, 41.59, 40.69, 40.26, 39.96, 36.84, 35.22, 34.94, 31.77, 31.38, 30.15, 29.69, 28.79, 26.38, 26.20, 26.13, 25.80, 23.68, 20.92, 17.10, 16.46, 14.48 ppm, ¹⁹F nmr spectrum (CDCl₃, 254 MHz): 40.0 ppm; LRMS; M+419, M-HF 399

### Example 3: 3α-5β,22α,22α,25R-spirostane-3-amine

At room temperature, cobalt (II) bromide (15 mg, 0.67 mmol) was added to dry ethanol (30 ml), making a light blue solution. 2,2'-dipyridyl (30 mg, 0.19 mmol) was added, and the solution became orange and then yellow. After adding sodium borohydride (0.16g, 4.2mmol) the solution was dark blue. The 3 α-azido-5β,20α,22α,25R-spirostan (1.40g, 3.2 mmol) was added slowly (slight exothermic, steady effervescence) with stirring. After 2h more stirring the mixture was allowed to stand overnight. It was then quenched with. acetic acid until no bubbling was observed with further addition (the pH was 7.0-7.5). The mixture was concentrated in vacuo, the residue partitioned between ether and water and the aqueous layer extracted (3x) with ether. The combined extracts were washed with water and dried over MgSO₄. The solvent was removed in vacuo to give an off-white waxy solid (0.63g).

### Example 4: 3α-methylsulfonylamino-5β,20α,22α,25R-spirostane

Methanesulfonyl chloride (0.46g, 4.0 mmol) was added dropwise to a stirred solution of 3α-5β,20α,22α,25R-spirostane-amine (0.55g, 1.32 mmol) in dry pyridine (15 ml). The mixture was heated on a steam bath for 10 min and then stood at room temperature overnight. Most of the pyridine was removed in vacuo, the oily residue partitioned between water (50 ml) and ether (50 ml) and the aqueous layer further extracted with ether (2x). The combined organic phases were washed with water and dried over MgSO₄. The solvent was removed in vacuo to give a tan solid (0.51g). Recrystallisation from acetone (2x) gave 0.27g of the above sulfonamide as white crystals, mp 201-204 **°** C.
m/z 493 (M⁺ for C₂₈H₄₇NSO₄). δ_{H} (270MHz, CDCl₃) 0.75 (3H, s, 18-CH₃), 0.79 (3H, d, *J*= 5.9 Hz, 27-CH₃), 0.95 (3H, 19-CH₃), 0.96 (3H, d, *J*= 7.3 Hz, 21-CH₃), 1.0-2.05 (27H, complex m, aliphatic H), 2.98 (3H,s. SMe), 3.20-3.55 (3H, complex m, 26-OCH₂, H-3), 4.40 (1H, br dd, *J*= 7.4, 8.9 Hz, H-16), 4.58 (1H, brd, *J*= 7.3 Hz, NH, exchang.). δ_{C} (270 MHz, CDCl₃) 14.5, 16.4, 17.1, 20.6,23.5,26.6,26.9, 28.8, 29.2, 303, 31.4, 31.8, 34.6, 35.0, 35.4, 35.9, 40.1, 40.6, 40.7, 41-6, 42.1, 42.5, 54.1 (C-3), 56.3 (C-14), 62.3 (C-17), 66.8 (C-26), 80.9 (C-16), 109.2 (C-22); R_{f} 0.3 (silica, ethyl acetate-hexane. 1:3)

### Example 5 : 3,3 difluoro-5β,20α,22α,25R-spirostane

To a solution of smilagenone (200 mg) in trichlorofluoromethane (10 ml) and DCM (5 ml) at O°C was added slowly (diethylamino)sulphur trifluoride (DAST, 80 mg). The reaction was stirred at room temperature overnight. Water was carefully added to quench the reaction followed by DCM. The organic layer was washed with water, dried (MgSO₄) filtered and evaporated *in vacuo.* The crude material was purified by column chromatography using ethyl acetate/petroleum ether (1:19) as eluent to afford 3,3 difluoro-5β,20α,22α,25R-spirostane as a white powder, TLC-. (silica gel) 10%Et)Ac/petrol R_{f} 0.45; melting point: 195-197°; ¹H nmr spectrum (CDCl₃, 500 MHz): 4.40 (1H,q) 3.50 (1H,dd), 3.40 (1H,t), 1.0 (3H,s), 0.98 (3H,d) 0.80 (3H,d)0.78 (3H,s); ¹³C nmr spectrum (CDCl₃, 125 MHz): . 109.27, 80.83, 66.89, 62.25, 56.29, 41.66, 40.69, 40.10, 39.96, 35.10, 34.72, 34.50, 34.18, 32.95, 31.77, 31.41, 30.30, 29,71, 29.32, 28.83, 26.31, 26.01, 22.81, 20.88, 17.14,16.46, 14.52 ppm

### Comparative Example 1:

Sarsasapogenin or smilagenin were oxidized using a mixture of sodium nitrite, boron trifluoro etherate and acetic acid to form the corresponding 23 keto-3-acetate compounds. Reaction with either chloranil or 2,3-dichloro,5,6-dicyano-1,4-benzoquinone (DDQ) afford the same 24,25 unsaturated ketone, which when reacted with lithium dimethyl copper in a Michael reaction affords, after deprotection, 25-methyl-5 β,20α,22α-spirostan-3β-ol.

### Biological results

Substituted sapogenins of the present invenion have also been tested for activity in a number of in-vitro assays. The assays/experiments that were considered of key importance in determining possible activity in the elevation of membrane bound receptor numbers were as follows:
Chinese hamster ovary (CHO) cells transfected with the a DNA fragment coding for a muscarinic receptor. The cell line used for the majority of the experiments was a cell line expressing the m2 receptor.

The methods and the results of these experiments are now described in turn.

### CHO cell line experiments

The effects of various compounds on the expression of m2 receptors on CHO cells transfected with DNA for the m2 receptor were investigated. Receptor numbers were assayed using tritiated QNB binding and subtracting non-specific binding. Compounds were dissolved in DMSO and DMSO was used as a control.
Compounds were tested at a range of final concentrations. Compounds were also tested in the presence and absence of tamoxifen to try to distinguish an oestrogen receptor mediated mechanism.

The results are summarised in the Table 1 below.

**Table 1. Effects of substituted sapogenins on the expression of m₂ receptors on CHO cells**

| Compound | Effect on receptor expression |
|---|---|
| 3β-3-fluoro-5β, 20α,22α,25R-spirostane | Active |
| 3α-fluoro-5α, 20α,22α,25R-spirostane | inactive |
| 3,3-difluoro-5β, 20α,22α,25R-spirostane | Active |

| 3α-methylsulphonylamino-5β,20α,22α,25R- pirostane | Active |
|---|---|
| 3α-azido-5β, 20α,22α,25R-spirostane | Active |
| 3α-amino-5β,20α,22α,25R-spirostane | Active |
| 25-methyl-5β,20α,22α-spirostan-3β-ol | Active |

"active" means increase over the control, "inactive" means same effect as control.

Thus the experiment indicates that several of the substituted sapogenins of the invention were able to increase the number of muscarinic receptors expressed on the surface of CHO cells cultured in-vitro. The effect was not antagonised by tamoxifen, indicating that the mechanism involved did not involve the oestrogen receptor.

It is speculated here that the effect of the active compounds claimed in this patent may operate through an effect on G protein and that the effects on receptor numbers are secondary to an effect on G-protein. When a membrane bound G-protein linked receptor is stimulated two basic sets of events are initiated: the effecter response; and the internalisation of the receptor. The subsequent processing of the receptor to the state where it is again in a form on the cell surface or other membrane surface where it can interact with another receptor ligand appears to be subject to a number of factors. A number of these factors or mechanisms appear to be G-protein linked. There is evidence that activation of m₃ receptors may have an effect on G-protein expression or levels. It is speculated that the actions of the compounds described in this patent may due to an interaction in the processes of receptor regeneration, G-protein linkage or G-protein homeostasis.

An alternative hypothesis is that the compounds are increasing the synthesis or release or a decreased rate of degradation of neurotropic factors such as brain derived growth factor and/or nerve growth factor. These effects on growth factors might be due to an effect of the compound on a cytosolic or nuclear receptor or the binding of a compound to a promoter region with a consequent effect directly on the rate of production of mRNA for the growth factor or as a consequence of increasing the production of another material factor such as G-protein or finally the effects may be secondary to an effect on receptor or G-protein procession.

The increased expression and/or abnormal processing of the amyloid precursor protein (APP) is associated with the formation of amyloid plaques and cerebrovascular amyloid deposits which are the major morphological hallmarks of Alzheimer's disease. Of particular interest are the processes regulating the proteolytic cleavage of APP into amyloidogenic and nonamyloidogenic fragments. The cleavage of APP by the enzyme α-secretase within the β-amyloid sequence of the protein results in the formation of a non amyloidogenic C-Terminal fragment, and the soluble APPsα fragment; this latter fragment has been shown to have neurotropic and neuroprotective activity as well as to enhance memory in mice when injected intra-cerebro-ventrically (ICV). In contrast, processing of APP by β-secretase exposes the N-terminus of β-amyloid which is released by γ-secretase cleavage at the variable C-terminus. The resulting β-amyloid peptides, which contain 39-43 amino acids, have been shown to be neurotoxic and to accumulate in plaques which interfere with inter-neurone connections.

A number of studies have shown that stimulation of the protein-kinase (PKC) linked muscarinic M₁ and M₃ receptors results in an increase in α-secretase activity,. As a consequence processing of APP to APPsα with its neuroprotective effects is increased. In parallel, processing of APP by β- and γ-secretase is decreased and there is a consequential reduction of β-amyloid. Other transmitters such as nerve growth factor (NGF) and brain derived neurotropic factor (BDNF) as well as bradykinin and vasopressin may have similar effects in increasing the proportion of APP processed to APPsα. There may be a number of factors involved in the effects of NGF which may include binding of the factor to the tyrosine kinase receptor (TrkA) and the stimulation of phospholipase Cγ with subsequent phosphorylation and activation of protein kinase C (PKC) and increase in relative activity of α-secretase.

Any treatment which increases activity of protein-kinase C selectively in brain might therefore be expected to be of use in the management of Alzheimer's disease. Until recently agonists selective at the M₁ receptor have not been available. Non-selective agonists would be expected to stimulate pre-synaptic M₂ receptors which cause negative feedback and hence would further severely impair muscarinic transmission. Selective agonists at the M₁ receptor are now becoming available (talsaclidine) and such agents are under investigation for the treatment of AD. There is however, a substantial risk that, as with the chronic administration of any receptor agonist, the clinical benefits seen will be severely limited in terms of the size of benefit by reducing receptor numbers or reducing sensitivity and in terms of side effects due to lack of receptor specificity. Thus compounds as described in this invention, which selectively increase muscarinic M₁ receptor numbers, with little or no effect on muscarinic M₂ receptor numbers in the brain would be expected to be devoid of the problems seen with a muscarinic agonist and hence have particular utility. Indeed the benefits may be seen in three parts as follows.
1. A selective increase in M₁ receptor numbers leading to increased synaptic transmission. Chronic administration of a selective agonist will, at best, have no adverse effect on transmission;
2. Secondary to the increased receptor numbers, an increase stimulation of PKC with a consequential increase in α-secretase activity, leading to:
2.1 A reduced production of β-amyloid and a consequent reduction of plaque formation and neuronal loss;
2.2 An increase in APPsα and a consequent improvement in cerebral function as witnessed by an improvement in short and long term memory.

The following Example is provided to illustrate further the invention in a non-limiting manner.

In a CHO cell line expressing recombinant human muscarinic receptors *in vitro,* the number of muscarinic receptors tends to decline with time. Substituted sapogenins of the invention (1-10µM) incubated for 72 hours increase muscarinic receptor density.

### Methods:

Effect of substituted sapogenins of the invention on muscarinic receptor density in CHO cells expressing recombinant human muscarinic receptors.

Chinese hamster ovary (CHO) cells expressing high levels of receptor (~2.2 pmoles receptor/mg protein) were cultured in flasks (150 ml) for 24 hours before the start of the experiments. Vehicle (DMSO) and substituted sapogenins (at 1 and 10 µM) were added to the medium for 48 h. The culture medium was discarded, the cells scraped off and resuspended in Hanks solution, centrifuged and m-receptor levels determined by incubating with [³H]-QNB for 30 min followed by liquid scintillation counting. Protein levels were determined by a micro Lowry method.

### Results:

Over the culturing period treatment with substituted sapogenins of the invention prevents the decrease in muscarinic receptor number in a concentration-dependent manner.

## Claims

1. A compound for use in treating cognitive dysfunction, chosen from (a) the compounds of general formula (I): 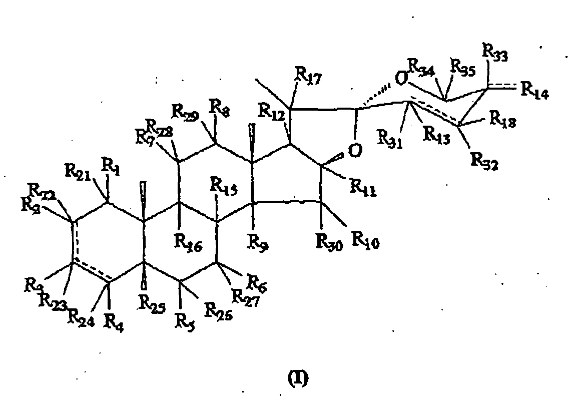 and their pharmaceutically acceptable salts,
wherein in the general fomula (I):
- R₁, R₂, R₄, R₅, R₆, R₇, R₈, R₉, R₁₀, R_{13,} R₁₈,R₂₁, R₂₂, R₂₃, R₂₅, R₂₆, R₂₇, R₂₈, R₂₉, R₃₀, R₃₁, R₃₂, R₃₄, R_{35,} are, independently of each other, either H, OH, =O and OR Where R = alkyl or acyl group or absent
- R_{9,} R₁₁, R₁₅, R₁₆, R₁₇, R₂₅ are either a H, OH, OR where R = alkyl or acyl group or absent;
- R₃₃, R₁₄ = H, alkyl group, OH, =O or OR when R = alkyl or acyl group or absent,
----- represents an optional double bond, and
wherein in addition to the above
R₃ is a X radical,
wherein X is chosen from the group consisting of:
-halo atom,
- (Me-S-), (Me-SO-), (Me-SO₂-),
- N₃- NH₂-, MeSO₂NH-, and
- alkyl;
(b) the compounds of general formula (II) : 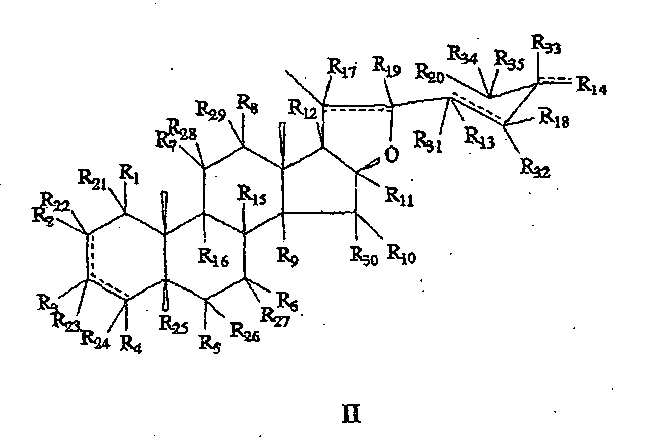 their pharmaceutically acceptable salts,
wherein in the general formula (II):
-R₁, R₂, R₄, R₅, R₆, R₇, R₈, R₁₀, R₁₃, R₁₈, R_{20,} R₂₁, R₂₂, R₂₃, R₂₄, R₂₅, R₂₇, R₂₉, R₂₉, R₃₁, R₃₂, B₃₄, R₃₅, are, independently of each other, either H, OH, =O, and OR where R= alkyl or acyl group or absent;
- R₉, R₁₁, R_{12,} R₁₅, R₁₆, R₁₇, R₁₉, R₂₅ are either a H, OH, OR where R = alkyl or acyl group or absent;
- R₃₃, R₁₄ = H, alkyl group, OH, =O or OR where R = alkyl or acyl group or absent,
represents an optional double bond, and
wherein in addition to the above
is a X radical,
wherein X is chosen from the group consisting of :
- halo atom,
• (Me-S-), (Me-SO-), (Me-SO₂-),
• N₃-, NH₂-, MeSO₂NH-, and
• alkyl; and
(c) the compounds of general formula (III): 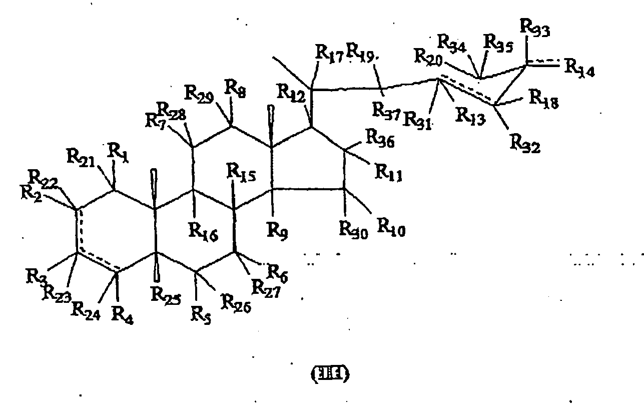 and their pharmaceutically acceptable salts,
R₁, R₂, R₄, R₅, R₆, R₇, R₈, R₁₀, R₁₃, R₁₈, R₁₉, R₂₀, R₂₁, R₂₂, R₂₃, R₂₄, R₂₆, R₂₇, R₂₈, R₂₉, R₃₀, R₃₁, R₃₂, R₃₄, R₃₅, R₃₆, R₃₇ are, independently of each other, either H, OH, =O, and OR where R = alkyl or acyl group or absent;
- R₉, R₁₁, R_{12,} R_{14,} R₁₅, R₁₆, R₁₇, R₂₅, R₃₃ can be either a H, OH, OR where R= alkyl or acyl group or absent;
-R₃₃, R₁₄= H, alkyl group, OH, OR where R= alkyl or acyl group or absent,
----- represents an optional double bond, and
wherein in addition to the above
R₃
is a X radical,
wherein X is chosen from the group consisting of:
• halo atom,
• (Me-S-), (Me-SO-), (Me-SO₂-),
• N₃-, NH₂-, MeSO₂NH-, and • alkyl, wherein the term "acyl" means a H-CO- or alkyl-co- group, and the term "alkyl" throughout means an aliphatic hydrocardon group which may be straight or branched having 1 to 20 carbon atoms in the chain.

2. A compound according to claim 1, wherein in the general formula (I):
R₁, R₂, R₄, R₅, R₆, R₇, R₈, R₁₀, R₁₃, R₁₈, R₂₁, R₂₂, R₂₃, R₂₄, R₂₆, R₂₇, R₂₈, R₂₉, R₃₀, R₃₁, R₃₂, R₃₄, R₃₅, are, independently of each other, either H, OH, =O, and OR where R = alkyl or acyl group or absent;
- R₉, R₁₂, R₁₅, R₁₆, R₁₇ =H,
- R₁₁, R₂₅, are either a H, OH, OR where R = alkyl or acyl group or absent;
- R₃₃, R₁₄ =H, alkyl group, OH, =O or OR where R ≃ alkyl or acyl group or absent,
represents an optional double bond, and
wherein in addition to the above
R3 is a radical,
wherein X is chosen from the group consisting of :
- halo atom,
• (Me-S-), -(Me-SO-), (Me-SO₂-),
• N₃-, NH₂-, MeSO₂NH-, and
• alkyl.

3. A compound according to claim 1, wherein in the general formula. (I):
-R₁= R₂= R₄= R₅=R₆=R₇=R₈= R₁₀=R₁₁= R₉= R₁₂=R₁₃=R₁₅=R₁₆,=R₁₇R₁₈= R₂₁=R₂₂=R₂₄=R₂₅=R₂₆=R₂₇=R₂₈=R₂₉=R₃₀=R₃₁=R₃₂=R₃₃=R₃₄=R₃₅=H
-R₁₄ =CH₃,
represents a single bond,
-the methyl group at C25 is either in the R or S configuration and
R₃ is a X radical, R₂₃ being H,
OH, =O, and OR where R = alkyl or acyl group or absent,
and X is chosen from the group consisting of :
**•** halo atom,
• (Me-S-), (Me-SO-), (Me-SO₂-), and
• N₃-, NH₂-, MeSO₂NH-, and
• alkyl.

4. A compound according to claim 1, wherein in the general formula (I):
-R₁=R₂=R₄=R₅=R₆=R₇=R₈=R₁₀=R₁₁=R₉=R₁₁=R₉=R₁₂=R₁₃R₁₅=R₁₇=R₁₈ R₂₁=R₂₂=R₂₄=R₂₅=R₂₆= R₂₈=R₂₉=R₃₀=R₃₁=R₃₂=R₃₄=R₃₅=H,
-R₁₄=R₃₃=alkyl,
represents a single bond,
R₃ is a X radical, R₂₃ being H,
OH, =O, and OR where R = alkyl or acyl group or absent
and X is chosen from the group consisting of:
• halo atom,
• (Me-S-), (Me-SO-), (Me-SO₂-), and
• N₃-, NH₂-, MeSO₂NH-, and
alkyl.

5. A compound according to claim 1, wherein in the general formula (II)
R₁, R₂, R₄, R₅, R_{6,} R₇, R₈, R₁₀, R₁₃, R₁₈, R₂₁, R₂₂, R₂₃, R₂₄, R₂₆, R₂₇, R₂₈, R₂₉, R₃₀, R₃₁, R₃₂, R₃₄, R₃₅, are, independently of each other, either H, OH, =O, and OR where R= alkyl or acyl group or absent;
-R₉, R₁₂, R₁₅, R₁₆, R₁₇ = H
- R₂₀= either H, OH, =O, and OR where R = alkyl, acyl or carbohydrate and
- R₁₁, R_{19,} R₂₅, are either a H, OH, OR where R = alkyl or acyl group or absent;
-R₃₃ R₁₄=H, alkyl group, OH, =O or OR where R= alkyl or acyl group or absent,
----- represents an optional double bond, and
wherein in addition to the above
R₃ is a X radical,
wherein X is chosen from the group consisting of :
• halo atom,
• (Me-S-). (Me-SO-), (Me-SO₂-).
• N₃-, NH₂-, MeSO₂NH-, and
• alkyl.

6. A compound according to claim 1, wherein in the general formula (II):
-R₁=R₂=R₄=R₅-R₆-R₇=R₈-R₁₀=R₁₁=R₉=R₁₂=R₁₃=R₁₅=R₁₆=R₁₇=R₁₈= R₂₁=R₂₂=R₂₃=R₂₄=R₂₅=R₂₆R₂₇=R₂₈R₂₉=R₃₀=R₃₁=R₃₂=R₃₃=R₃₄=R₃₅=H
-R₁₄ =CH₃,
- R₂₀= -OH or -OR where R = alkyl, acyl or carbohydrate and
R₁₉= H or is absent
----- represents an optional double bond, and
- the methyl group at C25 is either in the R or S configuration and
wherein in addition to the above
R₉ is a X radical, R₂₁ being H,
OH, =O, and OR where R= alkyl or acyl group or absent,
and X is chosen from the group consisting of ;
• halo atom,
• (Me-S-), (Me-SO-), (Me-SO₂-), and
• N₃-, NH₂-, MeSO₂NH-, and
• alkyl.

7. A compound according to claim 1, wherein in the general formula (II):
-R₁=R₂=R₄=R₅=R₆=R₇=R₈=R₁₀=R₁₁=R₉=R₁₂=R₁₃=R₁₅=R₁₆=R₁₇=R₁₈= R₂₁=R₂₂=R₂₃=R₂₄=R₂₅=R₂₆=R₂₇=R₂₃=R₂₉=R₃₀=R₃₁=R₃₂=R₃₄=R₃₅=H,
-R₁₄=R₃₃=alkyl,
- R₂₀= -OH or -OR where R = alkyl, acyl or carbohydrate and
R₁₉ = H or is absent
----- represents an optional double bond, and
wherein in addition to the above
R₃ is a X radical, R₂₃ being H, OH, =O, and OR where R = alkyl or acyl group or absent,
and X is chosen from the group consisting of :
• halo atom,
• (Me-S-), (Me-SO-), (Me-SO₂-), and
• N₃-, NH₂-, MeSO₂NH-, and
• alkyl.

8. A compound according to claim 1, wherein in the general formula (III)
- R₁, R₂, R₄, R₅, R₇, R₈, R₁₀, R₁₃, R₁₈, R₁₉, R₂₁, R₂₂, R₂₃, R₂₄, R₂₆, R₂₇, R₂₈, R₂₉,
R₃₀, R₃₁, R₃₂, R₃₄, R₃₅, R₃₆, R₃₇ are, independently of each other, either H, OH, =O, and OR where R= alkyl or acyl group or absent;
-R₉, R₁₂, R₁₅, R₁₆, R₁₇=H,
- R₂₀ = H, OH, =O, and OR where R = alkyl, acyl or carbohydrate and
- R₁₁, R₂₅, are either a H, OH, OR where R = alkyl or acyl group or absent;
- R₃₃, R₁₄ = H, alkyl group, OH, OR where R = alkyl or acyl group or absent,
----- represents an optional double bond, and
wherein in addition to the above
R₃
is a X radical,
wherein X is chosen from the group consisting of :
• halo atom,
• (Me-S-), (Me-SO-), (Me-SO₂-),
• N₃-, NHz-, MeSO₂NH-, and
• alkyl.

9. A compound according to claim 1, wherein in the general formula (III):
-R₁=R₂=R₄=R₅=R₆=R₇=R₈=R₁₀=R₁₁=R₉=R₁₂=R₁₃=R₁₅=R₁₆=R₁₇=R₁₈= R₂₁= R₂₂=R₂₃=R₂₄=R₂₅=R₂₆=R₂₇=R₂₈=R₂₉=R₃₀=R₃₁=R₃₂=R₃₃=R₃₄=R₃₅=H,
-R₁₄ = CH₃,
-R₂₀= -OH or OR where R = alkyl, acyl or carbohydrate and
R₁₉ = H or is about
R₃₇ = H, -OH or =O
R₃₅= H or -OH
----- represents a single bond, and
- the methyl group at C25 may be either in the R or S configuration and wherein in addition to the above.
R₃ is a X radical, R₂₃ being H,
OH, =O, and OR where R = alkyl or acyl group or absent,
and X is chosen from the group consisting of:
• halo atom,
• (Me-S-), (Me-SO-), (Me-SO₂-), and
• N₃-, NH₂-, MeSO₂NH-,
• alkyl.

10. A compound according to claim 1, wherein in the general formula (III):
-R₁=R₂=R₄=R₅=R₆=R₇=R₈=R₁₀=R₁₁=R₉=R₁₂=R₁₃=R₁₅=R₁₆=R₁₇=R₁₈= R₂₁=R₂₂=R₂₃=R₂₄=R₂₅=R₂₆=R₂₇=R₂₈=R₂₉=R₃₀=R₃₁=R₃₂=R₃₄=R₃₅=H,
-R₁₄=R₃₃=alkyl,
- R₂₀ =OH or -OR where R = alkyl, acyl or carbohydrate and
R₁₉=H or is absent
R₃₇=H, -OH or =O
R₃₆= H or OH
----- represents a single bond, and
wherein in addition to the above
R₃ is a X radical, R₂₃ being H,
OH=O, and OR where R = alkyl or acyl group or absent,
and X is chosen from the group consisting of:
- halo atom,
• (Me-S-), (Me-SO-), (Me-SO₂-), and
• N₃-,NH₂-, MeSO_{2N}H-,
• alkyl.

11. A compound according to any one of the preceding claims wherein in the definition of X, the halo atom is fluoro atom.

12. A compound chosen from : (3β-fluoro-5β, 20α,22α,25R-spirostane), (3,3-difluoro-5β,20α,22α,25R-spirostane), (3α-methlsulfonylamino-5β,20α,25R-spirostane), (3α-azido-5β, 20α,22α,25R-spirostane) and (3α-amino-5β,20α,22α,25R-spirostane), for use in treating cognitive dysfunction.

13. A compound according to claim 1, wherein the compound of general formula (I) is chosen from sarsasapogenin, episarsasapogenin, smitagemin and epismilagenin, in each case substituted at the R₃ site by X as defined in claim 1.

14. A compound according to any one of claims 1(b), 4, 5 and 7, wherein: -R₁₄ = R₃₃ = methyl

15. A compound according to any one of the preceding claims, for the treatment of Alzheimer's disease or a senile dementia of the Alzheimer's type, Parkinson's disease, Lewi body dementia, postural hypotension, autism, chronic fatigue syndrom, Myasthenia Gravis, Lambert Eaton disease, diseases and problems associated with Gulf war syndrome, occupational exposure to organophosphorus compounds and problems associated with ageing.

16. A compound according to claim 15, for the treatment of Alzheimer's disease or a senile dementia of the Alzheimer's type.

17. A medicament is for treating cognitive dysfunction, comprising a compound according to any one of claims 1 to 16.

18. The medicament as claimed in claim 17, wherein said medicament is for enhancing cognitive function in a patient suffering from ange-related dysfunction.

19. A food product or beverage to enhance cognitive fonction comprising a compound according to any one of claims 1 to 14.

20. A non-therapeutical method of enhancing cognitive function which comprises administering one or more of the compounds as defined in any one of claims 1 to 4.

## Patentansprüche

1. Verbindung zur Verwendung bei der Behandlung von kognitiver Dysfunktion, ausgewählt aus (a) den Verbindungen der allgemeinen Formel (I) und deren pharmazeutisch akzeptablen Salzen: 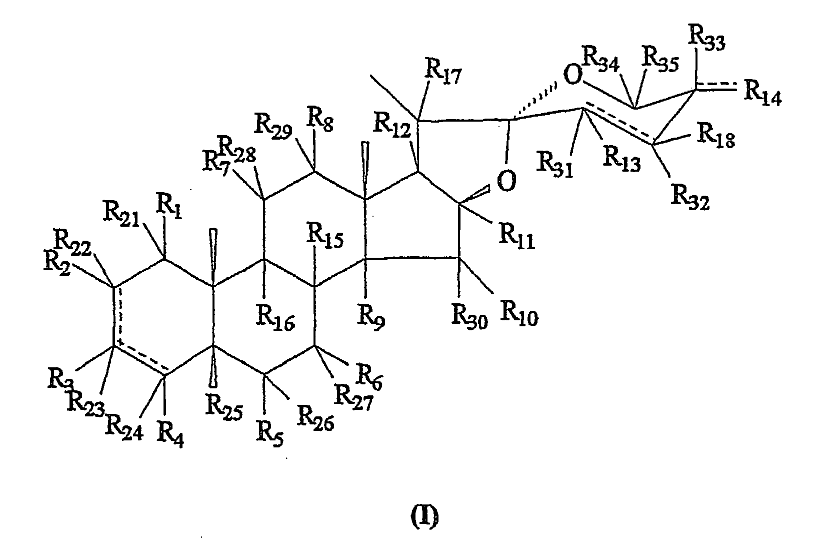 wobei in der allgemeinen Formel (I):
-R₁, R₂, R₄, R₅, R₆, R₇, R₈, R_{10,} R₁₃, R₁₈, R₂₁, R₂₂, R₂₃, R₂₅, R₂₆, R₂₇, R₂₈, R₂₉, R₃₀, R₃₁, R₃₂, R₃₄, R₃₅ unabhängig voneinander entweder H, OH, =O und OR darstellen,
wobei R= Alkyl- oder Acylgruppe ist oder fehlt;
-R₉, R₁₁, R₁₂, R₁₅, R₁₆, R₁₇, R₂₅ entweder H, OH, OR darstellen,
wobei R= Alkyl- oder Acylgruppe ist oder fehlt;
-R₃₃, R₁₄ = H, Alkylgruppe, OH, =O oder OR darstellen,
wobei R= Alkyl- oder Acylgruppe ist oder fehlt,
------- eine optionale Doppelbindung darstellt, und
wobei es sich zusätzlich zu dem Vorstehenden bei
- R₃ um ein X-Radikal handelt,
wobei X aus der Gruppe umfassend:
- Halogenatom,
- (Me-S-), (Me-SO-), (Me-SO₂-),
- N₃-, NH₂-, MeSO₂NH- und
- Alkyl ausgewählt ist;
(b) den Verbindungen der allgemeinen Formel (II): 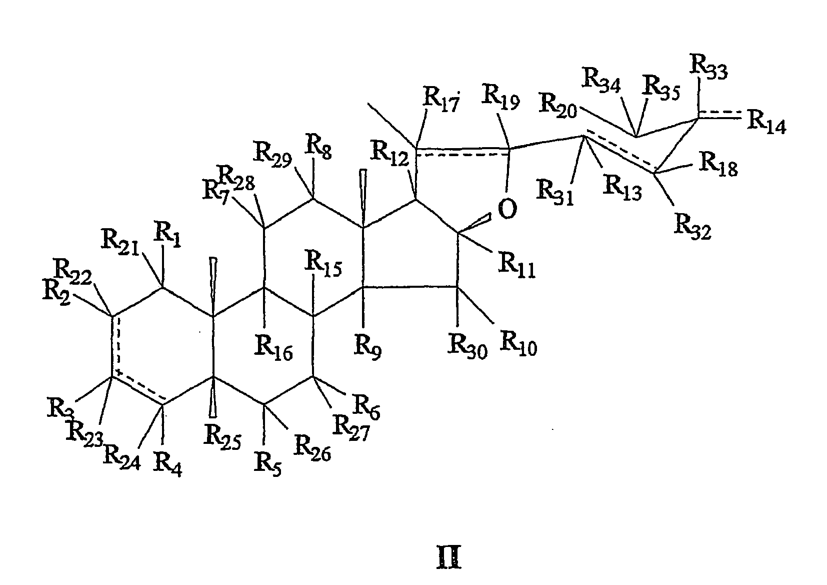 und deren pharmazeutisch akzeptablen Salzen,
wobei in der allgemeinen Formel (II):
- R₁, R₂, R₄, R₅, R₆, R₇, R₈, R₁₀, R₁₃, R₁₈, R₂₀, R₂₁, R₂₂, R₂₃, R₂₄, R₂₆, R₂₇, R₂₈, R₂₉, R₃₀, R₃₁, R_{32,} R₃₄, R₃₅ unabhängig voneinander entweder H, OH, =O, OR darstellen,
wobei R= Alkyl- oder Acylgruppe ist oder fehlt;
- R₉, R₁₁, R₁₂, R₁₅, R₁₆, R₁₇, R₁₉, R₂₅ entweder H, OH, OR darstellen,
wobei R= Alkyl- oder Acylgruppe ist oder fehlt;
- R₃₃ = R₁₄ = H, Alkylgruppe, OH, =O oder OR darstellen,
wobei R= Alkyl- oder Acylgruppe ist oder fehlt,
------ eine optionale Doppelbindung darstellt, und
wobei es sich zusätzlich zu dem Vorstehenden bei
R₃ um ein X-Radikal handelt,
wobei X aus der Gruppe umfassend:
- Halogenatom,
- (Me-S-), (Me-SO-), (Me-SO₂-),
- N₃-, NH₂-, MeSO₂NH- und
- Alkyl ausgewählt ist, und
(c) den Verbindungen der allgemeinen Formel (III): 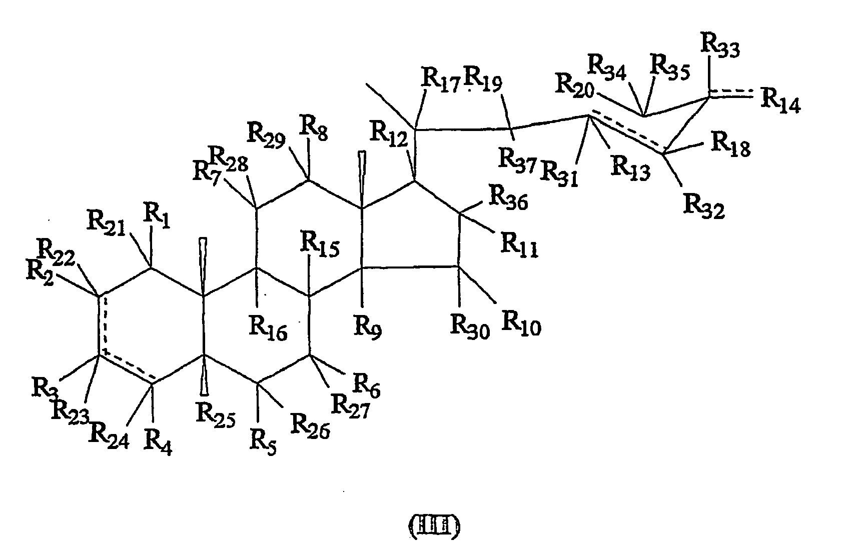 und deren pharmazeutisch akzeptablen Salzen,
wobei in der allgemeinen Formel (III):
- R₁, R₂, R₄, R₅, R₆, R₇, R₈, R₁₀, R₁₃, R₁₈, R₁₉, R₂₀, R₂₁, R₂₂, R₂₃, R₂₄, R₂₆, R₂₇, R_{28,}R₂₉, R₃₀, R₃₁, R₃₂, R₃₄, R₃₅, R₃₆ R₃₇ unabhängig voneinander entweder H, OH, =O, und OR darstellen,
wobei R= Alkyl- oder Acylgruppe ist oder fehlt;
- R₉, R₁₁, R₁₂, R₁₄, R₁₅, R₁₆, R₁₇, R₂₅, R₃₃ entweder H, OH, OR darstellen,
wobei R= Alkyl- oder Acylgruppe ist oder fehlt,
- R₃₃ = R₁₄ = H, Alkylgruppe, OH, OR darstellen,
wobei R= Alkyl- oder Acylgruppe ist oder fehlt,
------ eine optionale Doppelbindung darstellt, und
wobei es sich zusätzlich zu dem Vorstehenden bei
R₃ um ein X-Radikal handelt,
wobei X aus der Gruppe umfassend
- Halogenatom,
- (Me-S-), (Me-SO-), (Me-SO₂-),
- N₃-, NH₂-, MeSO₂NH- und
- Alkyl ausgewählt ist,
wobei sich der Begriff "Acyl" auf eine H-CO- oder Alkyl-CO-Gruppe bezieht und sich der Betriff "Alkyl" durchgängig auf eine unverzweigte oder verzweigte, in der Kette 1 bis 20 Kohlenstoffatome enthaltende, aliphatische Kohlenwasserstoffgruppe bezieht.

2. Verbindung nach Anspruch 1,
wobei in der allgemeinen Formel (I):
- R₁, R₂, R₄, R₅, R₆, R₇, R₈, R₁₀, R₁₃, R₁₈, R₂₁, R₂₂, R₂₃, R₂₄, R₂₆, R₂₇, R₂₈, R₂₉, R₃₀, R₃₁, R₃₂, R₃₄, R₃₅ unabhängig voneinander entweder H, OH, =O, und OR darstellen,
wobei R= Alkyl- oder Acylgruppe ist oder fehlt;
- R₉, R₁₂, R₁₅, R₁₆,R₁₇, =H,
- R₁₁, R₂₅ entweder H, OH, OR darstellen,
wobei R = Alkyl- oder Acylgruppe ist oder fehlt;
- R₃₃, R₁₄ =H, Alkylgruppe, OH, =O oder OR darstellen,
wobei R = Alkyl- oder Acylgruppe ist oder fehlt,
------ eine optionale Doppelbindung darstellt, und
wobei es sich zusätzlich zu dem Vorstehenden bei
R₃ um ein X-Radikal handelt,
wobei X aus der Gruppe umfassend:
- Halogenatom,
- (Me-S-), (Me-SO-), (Me-SO₂-),
- N₃-, NH₂-, MeSO₂NH- und
- Alkyl ausgewählt ist.

3. Verbindung nach Anspruch 1,
wobei in der allgemeinen Formel (I):
- R₁ = R₂ = R₄ = R₅ = R₆ = R₇ = R₈ = R₁₀ = R₁₁ R₉ = R₁₂ = R₁₃ = R₁₅ = R₁₆ = R₁₇ = R₁₈ =R₂₁ = R₂₂ = R₂₄ = R₂₅ = R₂₆ = R₂₇ = R₂₈ = R₂₉ = R₃₀ = R₃₁ = R₃₂ = R₃₃ R₃₄ = R₃₅ H, - R₁₄ = CH₃ darstellen,
------ eine Einfachbindung darstellt,
- die Methylgruppe an C25 entweder in der R- oder S-Konfiguration vorliegt, und
es sich bei R₃ um ein X-Radikal handelt,
- R₂₃ H, OH, =O und OR darstellt,
wobei R= Alkyl- oder Acylgruppe ist oder fehlt,
und X aus der Gruppe umfassend:
- Halogenatom,
- (Me-S-), (Me-SO-), (Me-SO₂-) und
- N₃-, NH₂-, MeSO₂NH- und
- Alkyl ausgewählt ist.

4. Verbindung nach Anspruch 1,
wobei in der allgemeinen Formel (I):
- R₁ = R₂ = R₄ = R₅ = R₆ = R₇ = R₈ = R₁₀ = R₁₁ = R₉ = R₁₂ = R₁₃ = R₁₅ = R₁₆ = R₁₇ = R₁₈ = R₂₁ = R₂₂ = R₂₄ = R₂₅ = R₂₆ = R₂₇ = R₂₈ = R₂₉ = R₃₀ = R₃₁ = R₃₂ = R₃₄ = R₃₅ = H,
- R₁₄ = R₃₃ = Alkyl darstellen,
------ eine Einfachbindung darstellt,
es sich bei R₃ um ein X-Radikal handelt,
- R₂₃ H, OH, =O, und OR darstellt,
wobei R= Alkyl- oder Acylgruppe ist oder fehlt,
und X aus der Gruppe umfassend:
- Halogenatom,
- (Me-S-), (Me-SO-), (Me-SO₂-) und
- N₃-, NH₂-, MeSO₂NH- und
- Alkyl ausgewählt ist.

5. Verbindung nach Anspruch 1,
wobei in der allgemeinen Formel (II):
- R₁, R₂, R₄, R₅, R₆, R₇, R₈, R₁₀, R₁₃, R₁₈, R₂₁, R₂₂, R₂₃, R₂₄, R₂₆, R₂₇, R₂₈, R₂₉, R₃₀, R₃₁, R₃₂, R₃₄, R₃₅ unabhängig voneinander entweder H, OH, =O und OR darstellen,
wobei R = Alkyl- oder Acylgruppe ist, oder fehlt;
- R₉, R₁₂, R₁₅, R₁₆, R₁₇ = H,
- R₂₀ = entweder H, OH, =O und OR darstellen,
wobei R= Alkyl, Acyl oder Kohlenhydrat ist, und
- R₁₁, R_{19,} R₂₅ entweder H, OH, OR darstellen,
wobei R= Alkyl- oder Acylgruppe ist, oder fehlt;
- R₃₃, R₁₄ = H, Alkylgruppe, OH, =O, oder OR darstellen,
wobei R= Alkyl- oder Acylgruppe ist oder fehlt,
------ eine optionale Doppelbindung darstellt, und
wobei es sich zusätzlich zu dem Vorstehenden bei R₃ um ein X-Radikal handelt,
wobei X aus der Gruppe umfassend:
- Halogenatom,
- (Me-S-), (Me-SO-), (Me-SO₂-),
- N₃-, NH₂-, MeSO₂NH- und
- Alkyl ausgewählt ist.

6. Verbindung nach Anspruch 1,
wobei in der allgemeinen Formel (II):
- R₁ = R₂ = R₄ = R₅ = R₆ = R₇ = R₈ = R₁₀ = R₁₁ = R₉ = R₁₂ = R₁₃ = R₁₅ = R₁₆ = R₁₇ = R₁₈ = R₂₁ = R₂₂ = R₂₃ = R₂₄ = R₂₅ = R₂₆ = R₂₇ = R₂₈ = R₂₉ = R₃₀ = R₃₁ = R₃₂ = R₃₃ = R₃₄ = R₃₅ = H,
- R₁₄ = CH₉,
- R₂₀ = -OH oder -OR darstellen,
wobei R= Alkyl, Acyl oder Kohlenhydrat ist, und
- R₁₉ = H darstellt oder fehlt,
------ eine optionale Doppelbindung darstellt, und
die Methylgruppe an C25 entweder in der R- oder S-Konfiguration vorliegt, und
wobei es sich zusätzlich zu dem Vorstehenden bei
R₃ um ein X-Radikal handelt,
- R₂₃ H, OH, =O und OR darstellt,
wobei R= Alkyl- oder Acylgruppe ist oder fehlt,
und X aus der Gruppe umfassend:
- Halogenatom,
- (Me-S-), (Me-SO-), (Me-SO₂-) und
- N₃-, NH₂-, MeSO₂NH- und
- Alkyl ausgewählt ist.

7. Verbindung nach Anspruch 1,
wobei in der allgemeinen Formel (II):
- R₁ = R₂ = R₄ = R₅ = R₆ = R₇ = R₈ = R₁₀ = R₁₁ = R₉ = R₁₂ = R₁₃ = R₁₅ = R₁₆= R₁₇ = R₁₈ = R₂₁ = R₂₂ = R₂₃ = R₂₄ = R₂₅ = R₂₆ = R₂₇ = R₂₈ = R₂₉ = R₃₀ = R₃₁ = R₃₂ = R₃₄ = R₃₅ = H,
- R₁₄ = R₃₃ = Alkyl,
- R₂₀ = -OH oder -OR darstellen,
wobei R= Alkyl, Acyl oder Kohlenhydrat ist, und
-R₁₉ = H ist oder fehlt,
------ eine optionale Doppelbindung darstellt, und
wobei es sich zusätzlich zu dem Vorstehenden bei
R₃ um ein X-Radikal handelt,
- R₂₃ H, OH, =O und OR darstellt,
wobei R= Alkyl- oder Acylgruppe ist oder fehlt,
und X aus der Gruppe umfassend:
- Halogenatom,
- (Me-S-), (Me-SO-), (Me-SO₂-) und
- N₃-, NH₂-, MeSO₂NH- und
- Alkyl ausgewählt ist.

8. Verbindung nach Anspruch 1,
wobei in der allgemeinen Formel (III):
- R₁, R₂, R₄, R₅, R₆, R₇, R₈, R₁₀, R₁₃, R_{18,} R₁₉, R₂₁, R₂₂, R₂₃, R₂₄, R₂₆, R₂₇, R₂₈, R₂₉, R₃₀, R₃₁, R₃₂, R₃₄, R₃₅, R₃₆, R₃₇ unabhängig voneinander entweder H, OH, =O oder OR darstellen,
wobei R= Alkyl- oder Acylgruppe ist oder fehlt;
- R₉, R₁₂, R₁₅, R₁₆, R₁₇, = H,
- R₂₀ = H, OH, =O und OR darstellen,
wobei R= Alkyl, Acyl oder Kohlenhydrat ist, und
- R₁₁, R₂₅ entweder H, OH, OR darstellen,
wobei R= Alkyl- oder Acylgruppe ist oder fehlt;
- R₃₃, R₁₄ = H, Alkylgruppe, OH, OR darstellen,
wobei R= Alkyl- oder Acylgruppe ist oder fehlt,
------ eine optionale Doppelbindung darstellt, und
wobei es sich zusätzlich zu dem Vorstehenden bei R₃ um ein X-Radikal handelt,
wobei X aus der Gruppe umfassend:
- Halogenatom,
- (Me-S-), (Me-SO-), (Me-SO₂-),
- N₃-, NH₂-, MeSO₂NH- und
- Alkyl ausgewählt ist.

9. Verbindung nach Anspruch 1,
wobei in der allgemeinen Formel (III):
- R₁ = R₂ = R₄ = R₅ = R₆ = R₇ = R₈ = R₁₀ = R₁₁ = R₉ = R₁₂ = R₁₃ = R₁₅ = R₁₆ = R₁₇ = R₁₈ = R₂₁= R₂₂ =R₂₃= R₂₄= R₂₅ = R₂₆ = R₂₇ = R₂₈= R₂₉= R₃₀ =R₃₁ = R₃₂= R₃₃ = R₃₄ =- R₃₅ = H,
- R₁₄ = CH₃,
- R₂₀ = -OH oder -OR darstellen,
wobei R= Alkyl, Acyl oder Kohlenhydrat ist, und
- R₁₉= H ist oder fehlt,
- R₃₇= H, -OH oder =O,
- R₃₅= H oder -OH ist,
------ eine Einfachbindung darstellt, und
- die Methylgruppe an C25 entweder in der R- oder
S-Konfiguration vorliegt, und
wobei es sich zusätzlich zu dem Vorstehenden bei
R₃ um ein X-Radikal handelt,
- R₂₃ H, OH, =O und OR darstellt,
wobei R= Alkyl- oder Acylgruppe ist oder fehlt,
und X aus der Gruppe umfassend:
- Halogenatom,
- (Me-S-), (Me-SO-), (Me-SO₂-) und
- N₃-, NH₂-, MeSO₂NH-,
- Alkyl ausgewählt ist.

10. Verbindung nach Anspruch 1,
wobei in der allgemeinen Formel (III):
- R₁ = R₂ = R₄ = R₅ = R₆ = R₇ = R₈ = R₁₀ = R₁₁ = R₉ = R₁₂ = R₁₃ = R₁₅ = R₁₆ = R₁₇ = R₁₈ = R₂₁ = R₂₂ =R₂₃= R₂₄ = R₂₅ = R₂₆ = R₂₇ = R₂₈ = R₂₉ = _{R30} = R₃₁=R₃₂= R₃₄=R₃₅ = H,
- R₁₄ = R₃₃ = Alkyl,
- R₂₀= OH oder -OR darstellen,
wobei R = Alkyl, Acyl oder Kohlenhydrat ist, und
- R₁₉ = H ist oder fehlt
- R₃₇ = H, -OH oder =O,
- R₃₆ = H oder -OH ist,
------ eine Einfachbindung darstellt, und
wobei es sich zusätzlich zu dem Vorstehenden bei R₃ um ein X-Radikal handelt,
wobei R₂₃ H, OH, =O und OR darstellt,
wobei R= Alkyl- oder Acylgruppe ist oder fehlt,
und X aus der Gruppe umfassend:
- Halogenatom,
- (Me-S-), (Me-SO-), (Me-SO₂-),
- N₃-, NH₂-, MeSO₂NH- und
- Alkyl ausgewählt ist.

11. Verbindung nach einem der vorangehenden Ansprüche,
wobei es sich bei dem Halogenatom in der Definition von X um ein Fluoratom handelt.

12. Verbindung ausgewählt aus der Gruppe umfassend:
(3β -fluoro-5β, 20α,22α-25R-Spirostan), (3,3-difluoro-5β,20α,22α,25R-Spirostan), (3α-methylsulphonylamino-5β,20α,22α,25R-Spirostan), (3α-azido-5β,20α,22α,25R-Spirostan) und (3α-amino-5β,20α,22α,25R-Spirostan) zur Verwendung bei der Behandlung von kognitiver Dysfunktion.

13. Verbindung nach Anspruch 1,
wobei die Verbindung der allgemeinen Formel (I) aus der Gruppe umfassend: Sarsasapogenin, Episarsasapogenin, Smilagenin und Epismilagen ausgewählt ist, wobei in jedem Fall die Stelle von R₃, wie im Anspruch 1 definiert, durch X substituiert ist.

14. Verbindung nach einem der vorangehenden Ansprüche 1(b), 4, 5 und 7,
wobei
- R₁₄ = R₃₃ = Methyl.

15. Verbindung nach einem der vorangehenden Ansprüche,
wobei diese zur Behandlung von Alzheimer-Krankheit oder Altersdemenz vom Alzheimer-Typ, Parkinson-Krankheit, Lewi-Körperchen-Demenz, orthostatischer Hypotonie, Autismus, chronischem Erschöpfungssyndrom, Myasthenia gravis, Lambert-Eaton-Syndrom, Erkrankungen und Beschwerden im Zusammenhang mit dem Golfkriegsyndrom, beruflich bedingten Belastungen mit Organoschwefelverbindungen und altersbedingten Beschwerden verwendet wird.

16. Verbindung nach Anspruch 15,
wobei diese zur Behandlung der Alzheimer-Krankheit oder Altersdemenz vom Alzheimer-Typ verwendet wird.

17. Medikament zur Behandlung von kognitiver Dysfunktion, umfassend eine Verbindung gemäß einem der Ansprüche 1 bis 16.

18. Medikament nach Anspruch 17,
wobei das Medikament zur Verbesserung der kognitiven Funktionen eines an altersbedingter Dysfunktion leidenden Patienten dient.

19. Nahrungsmittel oder Getränk zur Verbesserung der kognitiven Funktionen, umfassend
eine Verbindung gemäß einem der Ansprüche 1 bis 14.

20. Nichttherapeutisches Verfahren zur Verbesserung der kognitiven Funktionen, umfassend die Verabreichung einer oder mehrerer der gemäß einem der Ansprüche 1 bis 14 definierten Verbindungen.

## Revendications

1. Composé utilisable pour traiter la dysfonction cognitive, choisi parmi
(a) les composés de formule générale (I) : 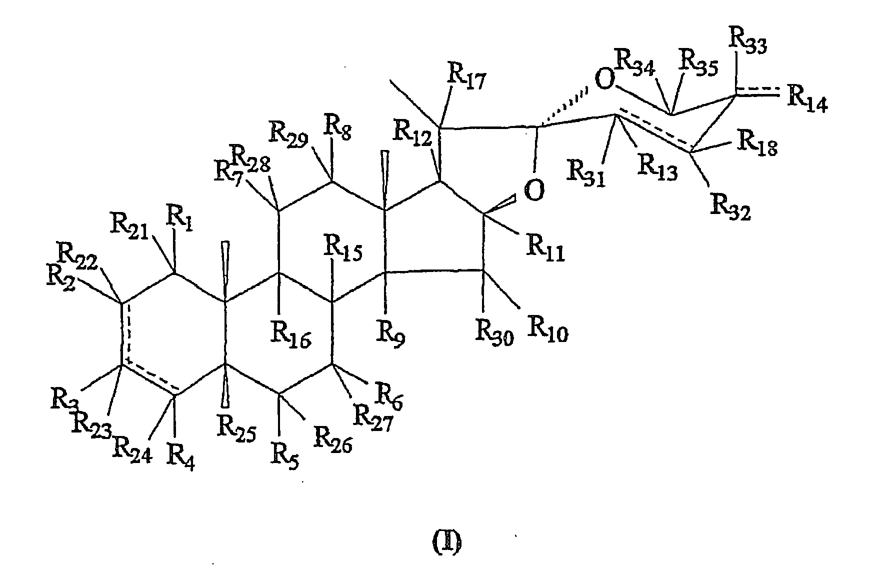 et leurs sels pharmaceutiquement acceptables,
dans lesquels, dans la formule générale (I) :
- R₁, R₂, R₄, R₅, R₆, R₇, R₈, R₁₀, R₁₃, R₁₈, R₂₁, R₂₂, R₂₃, R₂₄, R₂₆, R₂₇, R₂₈, R₂₉, R₃₀, R₃₁, R₃₂, R₃₄, R₃₅ sont, indépendamment les uns des autres, soit H, OH, =O, et OR où R ₌ groupe alkyle ou acyle ou absent ;
- R₉, R₁₁, R₁₂, R₁₅, R₁₆, R₁₇, R₂₅ sont soit H, OH, OR où R = groupe alkyle ou acyle ou absent ;
- R₃₃, R₁₄ = H, groupe alkyle, OH, =O ou OR où R ₌ groupe alkyle ou acyle ou absent,
------ représente une double liaison facultative, et dans lesquels, en plus de ce qui précède R₃ est un radical X,
où X est choisi dans le groupe constitué par :
- un atome d'halogène,
- (Me-S-), (Me-SO-), (Me-SO₂-),
- N₃-, NH₂-, MeSO₂NH-, et
- un groupe alkyle ;
(b) les composés de formule générale (II) : 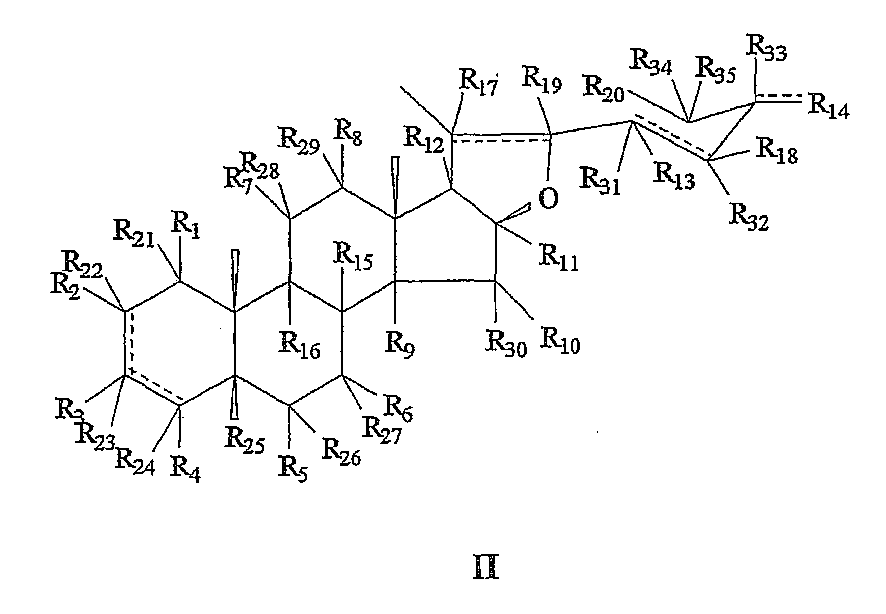 et leurs sels pharmaceutiquement acceptables,
dans lesquels, dans la formule générale (II) :
- R₁, R₂, R₄, R₅, R₆, R₇, R₈, R₁₀, R₁₃, R₁₈, R₂₀, R₂₁, R₂₂, R₂₃, R₂₄, R₂₆, R₂₇, R₂₈, R₂₉, R₃₀, R₃₁, R₃₂, R₃₄, R₃₅ sont, indépendamment les uns des autres, soit H, OH, =O, et OR où R = groupe alkyle ou acyle ou absent ;
- R₉, R₁₁, R₁₂, R₁₅, R₁₆, R₁₇, R₁₉, R₂₅ sont soit H, OH, OR où R = groupe alkyle ou acyle ou absent ;
- R₃₃, R_{14 =} H, groupe alkyle, OH, =O ou OR où R = groupe alkyle ou acyle ou absent,
------ représente une double liaison facultative, et
dans lesquels, en plus de ce qui précède
R₃ est un radical X,
où X est choisi dans le groupe constitué par :
- un atome d'halogène,
- (Me-S-), (Me-SO-), (Me-SO₂-),
- N₃-, NH₂-, MeSO₂NH-, et
- un groupe alkyle ; et
(c) les composés de formule générale (III) : 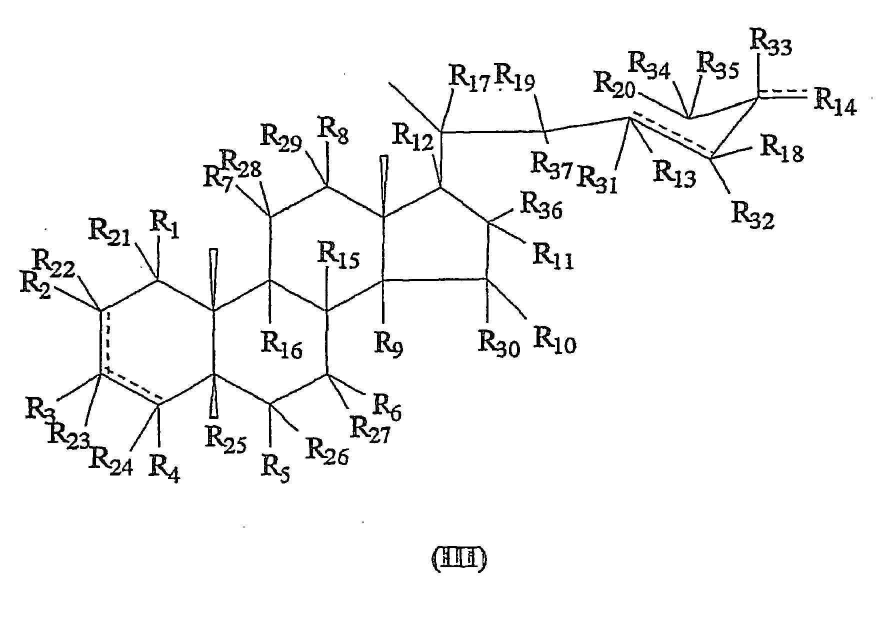 et leurs sels pharmaceutiquement acceptables,
dans lesquels, dans la formule générale (III) :
- R₁, R₂, R₄, R₅, R₆, R₇, R₈, R₁₀, R_{13,} R₁₈, R₁₉, R₂₀, R₂₁, R₂₂, R₂₃, R₂₄, R₂₆, R₂₇, R₂₈, R₂₉, R₃₀, R₃₁, R₃₂, R₃₄, R₃₅, R₃₆, R₃₇ sont, indépendamment les uns des autres, soit H, OH, =O, et OR où R = groupe alkyle ou acyle ou absent ;
- R₉, R₁₁, R₁₂, R₁₄, R₁₅, R₁₆, R₁₇, R₂₅, R₃₃ peuvent être soit H, OH, OR où R = groupe alkyle ou acyle ou absent ;
- R₃₃, R₁₄ = H, groupe alkyle, OH, OR où R = groupe alkyle ou acyle ou absent,
------ représente une double liaison facultative, et dans lesquels, en plus de ce qui précède
R₃ est un radical X,
où X est choisi dans le groupe constitué par :
- un atome d'halogène,
- (Me-S-) , (Me-SO-) , (Me-SO₂-),
- N₃-, NH₂-, MeSO₂NH-, et
- un groupe alkyle,
où le terme "acyle" désignant un groupe H-CO ou alkyl-CO, et le terme "alkyle" désignant un groupe hydrocarbure aliphatique qui peut être linéaire ou ramifié, ayant de 1 à 20 atomes de carbone dans la chaîne.

2. Composé selon la revendication 1, dans lequel dans la formule générale (I) :
- R₁, R₂, R₄, R₅, R₆, R₇, R₈, R₁₀, R₁₃, R₁₈, R₂₁, R₂₂, R₂₃, R₂₄, R₂₆, R₂₇, R₂₈, R₂₉, R₃₀, R₃₁, R₃₂, R₃₄, R₃₅ sont, indépendamment les uns des autres, soit H, OH, =0, et OR où R = groupe alkyle ou acyle ou absent ;
- R₉, R₁₂, R₁₅, R₁₆, R₁₇ = H,
- R₁₁, R₂₅ sont soit H, OH, OR où R = groupe alkyle ou acyle ou absent ;
- R₃₃, R₁₄ = H, groupe alkyle, OH, =O ou OR où R = groupe alkyle ou acyle ou absent,
------ représente une double liaison facultative, et dans lequel, en plus de ce qui précède
R₃ est un radical X,
où X est choisi dans le groupe constitué par :
- un atome d'halogène,
- (Me-S-), (Me-SO-), (Me-SO₂-),
- N₃-, NH₂-, MeSO₂NH-, et
- un groupe alkyle.

3. Composé selon la revendication 1, dans lequel dans la formule générale (I) :
- R₁ = R₂ = R₄ = R₅ = R₆ = R₇ = R₈ = R₁₀ = R₁₁ = R₉ = R₁₂ = R₁₃ = R₁₅ = R₁₆ R₁₇ = R₁₈ = R₂₁ = R₂₂ = R₂₄ = R₂₅ = R₂₆ = R₂₇ = R₂₈ = R₂₉ = R₃₀ = R₃₁ = R₃₂ = R₃₃ = R₃₄ = R₃₅ = H,
- R₁₄ = CH₃,
------ représente une liaison simple,
- le groupe méthyle en C25 est dans la configuration soit R, soit S et R₃ est un radical X, R₂₃ étant H, OH, =O, et OR où R = groupe alkyle ou acyle ou absent,
et X est choisi dans le groupe constitué par :
- un atome d'halogène,
- (Me-S-), (Me-SO-), (Me-SO₂-),
- N₃-, NH₂-, MeSO₂NH-, et
- un groupe alkyle.

4. Composé selon la revendication 1, dans lequel dans la formule générale (I) :
- R₁ = R₂ = R₄ = R₅ = R₆ = R_{7 =} R₈ = R₁₀ = R₁₁ = R₉ = R₁₂ = R₁₃ = R₁₅ = R₁₆ = R₁₇ = R₁₈ = R₂₁ = R₂₂ = R₂₄ = R₂₅ = R₂₆ = R₂₇ = R₂₈ = R₂₉ = R₃₀ = R₃₁ = R₃₂ = R₃₄ = R₃₅ = H,
- R₁₄ = R₃₃ = groupe alkyle,
------ représente une liaison simple,
- R₃ est un radical X, R₂₃ étant H, OH, =O, et OR où R = groupe alkyle ou acyle ou absent,
et X est choisi dans le groupe constitué par :
- un atome d'halogène,
- (Me-S-), (Me-SO-), (Me-SO₂-) ,
- N₃-, NH₂-, MeSO₂NH-, et
- un groupe alkyle.

5. Composé selon la revendication 1, dans lequel dans la formule générale (II) :
- R₁, R₂, R₄, R₅, R₆, R₇, R₈, R₁₀, R₁₃, R₁₈, R₂₁, R₂₂, R₂₃, R₂₄, R₂₆, R₂₇, R₂₈, R₂₉, R₃₀, R₃₁, R₃₂, R₃₄, R₃₅ sont, indépendamment les uns des autres, soit H, OH, =O, et OR où R = groupe alkyle ou acyle ou absent ;
- R₉, R₁₂, R₁₅, R₁₆, R₁₇ = H,
- R₂₀ = soit H, OH, =0, et OR où R = alkyle, acyle ou glucide et
- R₁₁, R₁₉, R₂₅ sont soit H, OH, OR où R = groupe alkyle ou acyle ou absent ;
- R₃₃, R_{14 =} H, groupe alkyle, OH, =0 ou OR où R = groupe alkyle ou acyle ou absent,
------ représente une double liaison facultative, et
dans lequel, en plus de ce qui précède
R₃ est un radical X,
où X est choisi dans le groupe constitué par :
- un atome d'halogène,
- (Me-S-) , (Me-SO-), (Me-SO₂-) ,
- N₃-, NH₂-, MeSO₂NH-, et
- un groupe alkyle.

6. Composé selon la revendication 1, dans lequel dans la formule générale (II) :
- R₁ = R₂ = R₄ = R₅ = R₆ = R₇ = R₈ = R₁₀ = R₁₁ = R₉ = R₁₂ = R₁₃ = R₁₅ = R₁₆ = R₁₇ = R₁₈ R₂₁ = R₂₂ = R₂₃ = R₂₄ = R₂₅ = R₂₆ = R₂₇ = R₂₈ = R₂₉ = R₃₀ = R₃₁ = R₃₂ = R₃₃ = R₃₄ = R₃₅ = H,
- R_{14 =} CH₃,
- R₂₀ = -OH ou -OR où R = alkyle, acyle ou glucide et
- R₁₉ = H ou est absent
------ représente une double liaison facultative, et
- le groupe méthyle en C25 est dans la configuration soit R, soit S et
dans lequel, en plus de ce qui précède
R₃ est un radical X, R₂₃ étant H, OH, =O, et OR où R = groupe alkyle ou acyle ou absent,
et X est choisi dans le groupe constitué par :
- un atome d'halogène,
- (Me-S-), (Me-SO-) , (Me-SO₂-),
- N₃-, NH₂-, MeSO₂NH-, et
- un groupe alkyle.

7. Composé selon la revendication 1, dans lequel dans la formule générale (II) :
- R₁ = R₂ = R₄ = R₅ = R₆ = R₇ = R₈ = R₁₀ = R₁₁ = R₉ = R₁₂ = R₁₃ = R₁₅ = R₁₆ = R₁₇ = R₁₈ = R₂₁ = R₂₂ = R₂₃ = R₂₄ = R₂₅ = R₂₆ = R₂₇ = R₂₈ = R₂₉ = R₃₀ = R₃₁ = R₃₂ = R₃₄ = R₃₅ = H,
- R₁₄ = R₃₃ = alkyle,
- R₂₀ = -OH ou -OR où R = alkyle, acyle ou glucide et
- R₁₉= H ou est absent ;
------ représente une double liaison facultative, et
dans lequel, en plus de ce qui précède
R₃ est un radical X, R₂₃ étant H, OH, =0, et OR où R = groupe alkyle ou acyle ou absent,
et X est choisi dans le groupe constitué par :
- un atome d'halogène,
- (Me-S-), (Me-SO-) , (Me-SO₂-) ,
- N₃-, NH₂-, MeSO₂NH-, et
- un groupe alkyle.

8. Composé selon la revendication 1, dans lequel dans la formule générale (III) :
- R₁, R₂, R₄, R₅, R₆, R₇, R₈, R₁₀, R₁₃, R₁₈, R₁₉, R₂₁, R₂₂, R₂₃, R₂₄, R₂₆, R₂₇, R₂₈, R₂₉, R₃₀, R₃₁, R₃₂, R₃₄, R₃₅, R₃₆, R₃₇ sont, indépendamment les uns des autres, soit H, OH, =O, et OR où R = groupe alkyle ou acyle ou absent ;
- R₉, R₁₂, R₁₅, R₁₆, R₁₇ = H,
- R₂₀ = H, OH, =O, et OR où R = alkyle, acyle ou glucide et
- R₁₁, R₂₅ sont soit H, OH, OR où R = groupe alkyle ou acyle ou absent ;
- R₃₃, R₁₄ = H, groupe alkyle, OH, OR où R = groupe alkyle ou acyle ou absent,
------ représente une double liaison facultative, et
dans lequel, en plus de ce qui précède
R₃ est un radical X,
où X est choisi dans le groupe constitué par :
- un atome d'halogène,
- (Me-S-), (Me-SO-), (Me-SO₂-),
- N₃⁻, NH₂⁻, MeSO₂NH-, et
- un groupe alkyle.

9. Composé selon la revendication 1, dans lequel dans la formule générale (III) :
- R₁ = R₂ = R₄ = R₅ = R₆ = R₇ = R₈ = R₁₀ = R₁₁ = R₉ = R₁₂ = R₁₃ R₁₅ = R₁₆ = R₁₇ = R₁₈ = R₂₁ = R₂₂ = R₂₃ = R₂₄ = R₂₅ = R₂₆ = R₂₇ = R₂₈ = R₂₉ = R₃₀ = R₃₁ = R₃₂ = R₃₃ = R₃₄ = R₃₅ = H,
- R₁₄ = CH₃,
- R₂₀ = -OH ou -OR où R = alkyle, acyle ou glucide et
- R₁₉ = H ou est absent ;
- R₃₇ = H, -OH ou =O
- R₃₆ = H ou -OH
------ représente une liaison simple, et
- le groupe méthyle en C25 peut être dans la configuration soit R, soit S et
dans lequel, en plus de ce qui précède
R₃ est un radical X, R₂₃ étant H, OH, =O, et OR où R = groupe alkyle ou acyle ou absent,
et X est choisi dans le groupe constitué par :
- un atome d'halogène,
- (Me-S-), (Me-SO-), (Me-SO₂-),
- N₃-, NH₂-, MeSO₂NH-, et
- un groupe alkyle.

10. Composé selon la revendication 1, dans lequel dans la formule générale (III) :
- R₁ = R₂ = R₄ = R₅ = R₆ = R₇ = R₈ = R₁₀ = R₁₁ = R₉ = R₁₂ = R₁₃ = R₁₅ = R₁₆ = R₁₇ = R₁₈ = R₂₁ = R₂₂ = R₂₃ = R₂₄ = R₂₅ = R₂₆ = R₂₇ = R₂₈ = R₂₉ = R₃₀ = R₃₁ = R₃₂ = R₃₄ = R₃₅ = H,
- R₁₄ = R₃₃ = alkyle,
- R₂₀ = -OH ou -OR où R = alkyle, acyle ou glucide et
- R₁₉ = H ou est absent
- R₃₇ = H, -OH ou =O
- R₃₆ = H ou -OH
⁻⁻⁻⁻⁻⁻ représente une liaison simple, et
dans lequel, en plus de ce qui précède
R₃ est un radical X, R₂₃ étant H, OH, =0, et OR où R = groupe alkyle ou acyle ou absent,
et X est choisi dans le groupe constitué par :
- un atome d'halogène,
- (Me-S-), (Me-SO-), (Me-SO₂-),
- N₃-, NH₂-, MeSO₂NH-, et
- un groupe alkyle.

11. Composé selon l'une quelconque des revendications précédentes, dans lequel dans la définition de X, l'atome d'halogène est un atome fluoro.

12. Composé choisi parmi :
(3β-fluoro-5β, 20α, 22α, 25R-spirostane), (3,3-difluoro-5β, 20α,-22α, 25R-spirostane), (3α-méthylsulfonylamino-5β, 20α, 22α, 25R-spirostane), (3α-azido-5β, 20α, 22α, 25R-spirostane) et (3α-amino-5β, 20α, 22α, 25R-spirostane), utilisable pour traiter la dysfonction cognitive.

13. Composé selon la revendication 1, dans lequel le composé de formule générale (I) est choisi parmi la sarsasapogénine, l'épisarsasapogénine, la smilagénine et l'épismilagénine, substituée dans chaque cas en R₃ par X, tel que défini dans la revendication 1.

14. Composé selon l'une quelconque des revendications 1(b), 4, 5 et 7, dans lequel :
- R₁₄ = R₃₃ = méthyle .

15. Composé selon l'une quelconque des revendications précédentes pour le traitement de la maladie d'Alzheimer ou d'une démence sénile du type Alzheimer, la maladie de Parkinson, la démence à corps de Lewy, l'hypotension posturale, l'autisme, le syndrome de fatigue chronique, la myasthénie auto-immune, la maladie de Lambert-Eaton, les maladies et les problèmes liés au syndrome de la guerre du Golfe, l'exposition professionnelle à des composés organophosphorés et les problèmes liés au vieillissement.

16. Composé selon la revendication 15 pour le traitement de la maladie d'Alzheimer ou d'une démence sénile du type Alzheimer.

17. Médicament pour traiter la dysfonction cognitive comprenant un composé selon l'une quelconque des revendications 1 à 16.

18. Médicament selon la revendication 17, dans lequel ledit médicament sert à renforcer la fonction cognitive chez un patient atteint de dysfonction liée à l'âge.

19. Produit alimentaire ou boisson permettant de renforcer la fonction cognitive comprenant un composé selon l'une quelconque des revendications 1 à 14.

20. Procédé non thérapeutique pour renforcer la fonction cognitive qui comprend l'administration d'un ou de plusieurs composés tels que définis dans l'une quelconque des revendications 1 à 14.
